(19) Europäisches Patentamt
European Patent Office
Office européen des brevets

(11) EP 1 824 827 B1

(12) EUROPÄISCHE PATENTSCHRIFT

(45) Veröffentlichungstag und Bekanntmachung des Hinweises auf die Patenterteilung:
**17.09.2008 Patentblatt 2008/38**

(21) Anmeldenummer: **05810848.1**

(22) Anmeldetag: **18.11.2005**

(51) Int Cl.:
***C07D 213/74*** (2006.01) ***C07D 233/58*** (2006.01)
***C07C 279/02*** (2006.01) ***C07D 295/037*** (2006.01)
***C07F 9/54*** (2006.01)

(86) Internationale Anmeldenummer:
**PCT/EP2005/012401**

(87) Internationale Veröffentlichungsnummer:
**WO 2006/063656 (22.06.2006 Gazette 2006/25)**

# (54) VERFAHREN ZUR HERSTELLUNG VON ONIUM-SALZEN MIT ALKYL- ODER ARYLSUFONAT-ANIONEN ODER ALKYL- ODER ARYLCARBOXYLAT-ANIONEN MIT GERINGEN HALOGENID-GEHALT

METHOD FOR PRODUCING ONIUM SALTS COMPRISING ALKYL ANIONS OR ARYL SULFONATE ANIONS OR ALKYL ANIONS OR ARYL CARBOXYLATE ANIONS HAVING A LOW HALIDE CONTENT

PROCEDE POUR PRODUIRE DES SELS D'ONIUM COMPRENANT DES ANIONS D'ALKYLE OU D'ARYLSULFONATE OU D'ALKYLE OU D'ARYLCARBOXYLATE PRESENTANT UNE TENEUR FAIBLE EN HALOGENURE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **14.12.2004 DE 102004060076**

(43) Veröffentlichungstag der Anmeldung:
**29.08.2007 Patentblatt 2007/35**

(73) Patentinhaber: **Merck Patent GmbH**
**64293 Darmstadt (DE)**

(72) Erfinder:
- **IGNATYEV, Nikolai (Mykola)**
  **47058 Duisburg (DE)**
- **WELZ-BIERMANN, Urs**
  **64646 Heppenheim (DE)**
- **KUCHERYNA, Andriy**
  **42117 Wuppertal (DE)**
- **WILLNER, Helge**
  **45481 Muelheim/Ruhr (DE)**

(56) Entgegenhaltungen:
**EP-A- 1 182 197** **WO-A-20/04106288**
**US-A- 2 585 979** **US-A- 5 532 411**

- **DARIO LANDINI, ANGELAMARIA MAIA, ALESSANDRO RAMPOLDI: JOURNAL OF ORGANIC CHEMISTRY, Bd. 54, 1989, Seiten 328-332, XP002366804**
- **KREITER R ET AL: "Design and synthesis of tris [bis(benzylammonium)aryl]phosphines with bulky meta-substituents" TETRAHEDRON, ELSEVIER SCIENCE PUBLISHERS, AMSTERDAM, NL, Bd. 59, Nr. 22, 26. Mai 2003 (2003-05-26), Seiten 3989-3997, XP004426769 ISSN: 0040-4020**
- **F. F. DUARTE, F. D. POPP: "Reissert Compound studies. LXIV. Reissert Compound and other products from an isolated N-benzoylisoquinolinium triflate salt" JOURNAL OF HETEROCYCLIC CHEMISTRY, Bd. 28, 1991, Seiten 1801-1804, XP002366805**
- **DARIO LANDINDI ET AL.: JOURNAL OF ORGANIC CHEMISTRY, Bd. 50, 1985, Seiten 117-118, XP002366806**
- **MARCO DE GIORGI: "A facile and convienent synthesis of lipophilic tetraalkylammonium or phosphonium hydrogen sulfates" SYNTHETIC COMMUNICATIONS, Bd. 17, Nr. 5, 1987, Seiten 521-533, XP009061293**
- **RADU BACALOGLU ET AL.: "Micellar Enhancements of Rates of SN2 Reactions of Halide Ions. The Effect of Headgroup Size" JOURNAL OF PHYSICAL CHEMISTRY, Bd. 93, 1989, Seiten 1497-1502, XP002366894**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 824 827 B1

- CLELIA R. A. BERTONCINI ET AL.: "Effect of 1-Butanol upon SN2 Reactions in Cationic Micelles. A Quantitative Treatment" JOURNAL OF PHYSICAL CHEMISTRY, Bd. 94, 1990, Seiten 5875-5878, XP002366895
- PETER J. STANG ET AL.: "Preparation of Nitrogen-Containig Bis(heteroaryl)iodonium Salts" SYNTHESIS, Bd. 8, 1995, Seiten 937-938, XP002366905
- SERGIO ALUNNI ET AL.: "The Hammett equation applied to the nucleophilic displacement of ions and ion pairs on substituted benzenesulphonates" JOURNAL OF PHYSICAL ORGANIC CHEMISTRY, Bd. 14, 2001, Seiten 265-270, XP009061502
- E. A. MEL' NICHUK ET AL.: "Reaction of Alkyldiiodiphosphines with Tertiary Amines" JOURNAL OF GENERAL CHEMISTRY USSR (ENGL. TRANS.), Bd. 55, Nr. 5, 1985, Seiten 896-899, XP009061514
- LANDINI D ET AL: "Anion nucleophilicity in ionic liquids: a comparison with traditional molecular solvents of different polarity" TETRAHEDRON LETTERS, ELSEVIER, AMSTERDAM, NL, Bd. 46, Nr. 23, 6. Juni 2005 (2005-06-06), Seiten 3961-3963, XP004881062 ISSN: 0040-4039
- JEFFRY P. WHITTEN ET AL.: "Cyanogen bromide-diemethylaminopyridine (CAP): A convenient source of positive cyanide for the synthesis of 2-cyanoimidazoles" SYNTHESIS, Bd. 6, 1988, Seiten 470-472, XP002366808

## Beschreibung

Die Erfindung betrifft ein Verfahren zur Herstellung von Onium-Salzen mit Alkyl- oder Arylsulfonat-Anionen oder Alkyl- oder Arylcarboxylat-Anionen durch Umsetzung eines Onium-Halogenids mit einem Alkyl- oder Trialkylsilylester einer Alkyl- oder Arylsulfonsäure oder einer Alkyl- oder Arylcarbonsäure oder deren Anhydride.

Eine große Anzahl von Onium-Salzen, insbesondere Alkyl- oder Arylsulfonate bzw. Alkyl- oder Arylcarboxylate, sind ionische Flüssigkeiten. Ionische Flüssigkeiten stellen durch Ihre Eigenschaften in der modernen Forschung eine wirksame Alternative zu traditionellen flüchtigen organischen Solventien für die organische Synthese dar. Die Verwendung von ionischen Flüssigkeiten als neues Reaktionsmedium könnte weiterhin eine praktische Lösung sowohl für die Lösungsmittel Emission als auch für Probleme in der Wiederaufbereitung von Katalysatoren sein.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf. Der Schmelzpunkt kann jedoch auch höher liegen, ohne die Anwendbarkeit der Salze auf allen Anwendungsgebieten zu beschränken. Beispiele für organische Kationen sind unter anderem Tetraalkylammonium, Tetraalkylphosphonium, N-Alkylpyridinium, 1,3-Dialkylimidazolium oder Trialkylsulfonium. Unter einer Vielzahl von geeigneten Anionen sind beispielsweise $BF_4^-$, $PF_6^-$, $SbF_6^-$, $NO_3^-$, $CF_3SO_3^-$, $(CF_3SO_2)_2N^-$, $ArylSO_3^-$, $CF_3CO_2^-$, $CH_3CO_2^-$ oder $Al_2Cl_7^-$ zu nennen.

Eine generelle Methode zur Herstellung von Onium-Sulfonaten bzw. - carboxylaten ist die Alkylierung der organischen Base, d.h. beispielsweise des Amins, Phosphins, Guanidins oder der heterocyclischen Base, mit Alkylestern einer Alkyl- oder Arylsulfonsäure, beispielsweise Alkyl-p-toluensulfonat (Alkyltosylat), bzw. einer Alkyl- oder Arylcarbonsäure, beispielsweise Alkyltrifluoracetat, auch bekannt durch (Richard C. Larock, Comprehensive Organic Transformations, 2nd Edition, Wiley-VCH, New York, 1999, oder P. Wasserscheid und T. Welton (Eds), Ionic Liquids in Synthesis, Wiley-VCH, 2003).

Ein Nachteil dieser Methode ist jedoch, dass ein Substituent des entstehenden Onium-Kations immer der entsprechenden Alkylgruppe des Alkylesters entspricht. Setzt man beispielsweise 1-Butylimidazolium mit Methyl-p-toluensulfonat um, so entsteht 1-Butyl-3-methylimidazolium p-Toluensulfonat. Erwünscht sind jedoch unsymmetrisch substituierte Onium-Salze, d.h. Salze, bei denen die Alkylgruppe des eingesetzten Esters nicht Substituent des entstehenden Onium-Salzes wird.

Unsymmetrische Onium-Salze mit Alkyl- oder Arylsulfonat-Anionen bzw. Alkyl- oder Arylcarboxylat-Anionen, wie zuvor definiert, können auch durch eine Metathese hergestellt werden, indem ein Oniumhalogenid mit einem entsprechenden Alkalimetallsalz der entsprechenden Säure umgesetzt würde. Das anfallende Alkalimetallhalogenid, z.B. Natriumchlorid, muss jedoch durch eine zusätzliche Aufreinigungsmethode entfernt werden. Die Verunreinigung durch Halogenid-Ionen, beispielsweise Chlorid-Ionen, größer als 1000 ppm (0,1 %), reduziert die Anwendbarkeit der ionischen Flüssigkeit, insbesondere in der Anwendung für elektrochemische Prozesse. Daher ist bei Verfahren zur Herstellung von Onium-Salzen, insbesondere ionischer Flüssigkeiten, die Technologie von entscheidender Bedeutung, damit diese durch die Reaktion per se oder die Reaktionsführung mit geringen Verunreinigungen synthetisiert werden können und so weitere kostenintensive zusätzliche Verfahrensschritte bei der Synthese entfallen.

Aufgabe der vorliegenden Erfindung war dementsprechend ein alternatives Verfahren zur Herstellung von Onium-Salzen mit Alkyl- oder Arylsulfonat-Anionen oder Alkyl- oder Arylcarboxylat-Anionen mit geringem Halogenid-Gehalt zur Verfügung zu stellen, welches zu Salzen, vorzugsweise von unsymmetrisch substituierten Onium-Salzen, mit hoher Reinheit in guter Ausbeute führt und auch für eine großtechnische Produktion geeignet ist.

Selbstverständlich ist ein solches Verfahren dann auch zur Herstellung von symmetrisch substituierten Onium-Sulfaten geeignet.

Die Aufgabe wird durch das erfindungsgemäße Verfahren gelöst, da der eingesetzte Alkyl- oder Trialkylsilylester der Sulfon- oder Carbonsäure oder das entsprechende Anhydrid das Anion des eingesetzten Oniumhalogenids alkyliert oder acyliert und nicht das organische Onium-Kation. Die als Nebenprodukt entstehenden Alkyl-, Trialkylsilyl- oder Acylhalogenide sind in der Regel Gase oder sehr flüchtige Verbindungen, die ohne großen prozesstechnischen Aufwand aus der Reaktionsmischung entfernt werden können. Einige dieser Nebenprodukte sind selbst wertvolle Materialien für organische Synthesen.

Gegenstand der Erfindung ist daher ein Verfahren zur Herstellung von Onium-Salzen mit Alkyl- oder Arylsulfonat-Anionen oder Alkyl- oder Arylcarboxylat-Anionen durch Umsetzung eines Onium-Halogenids mit einem Alkyl- oder Trialkylsilylester einer Alkyl- oder Arylsulfonsäure oder einer Alkyl- oder Arylcarbonsäure oder deren Anhydride. Erfindungsgemäß findet die Umsetzung des Onium-Halogenids mit einem Alkylester einer Sulfonsäure bei Raumtemperatur statt.

Aus US 2,585,979 ist die Herstellung von quaternären Sulfonaten von Pyrimidyl-aminochinolin-Derivaten

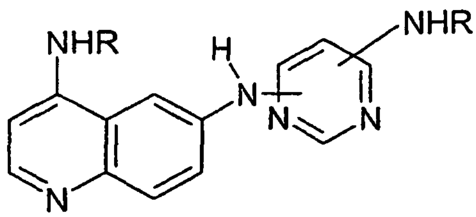

durch Umsetzung des entsprechenden Pyrimidyl-aminochinolin-Halogenids mit einem Niedrigalkylester einer Sulfonsäure bekannt. Die Reaktion mit Methylmethansulfonat findet jedoch im Gegensatz zum erfindungsgemäßen Verfahren bei Temperaturen von 130° bis 140°C in Gegenwart eines Lösungsmittels, beispielsweise einem Gemisch aus Nitrobenzen und Toluol, statt. Die Reaktion mit Methyl-p-toluensulfonat findet ohne Lösungsmittelzusatz bei ebenfalls hohen Temperaturen von 130° bis 135°C statt. Überraschenderweise gelingt jedoch die erfindungsgemäße Umsetzung mit den Alkylestern der Sulfonsäuren bei Raumtemperatur, d.h. bei Temperaturen zwischen 10° und 30°C, ohne Einsatz eines Lösungsmittels in annähernd quantitativer Ausbeute.
Ein Hinweis, dass Trialkylsilylester der Sulfon- oder Carbonsäure oder ein Anhydrid der Sulfon- oder Carbonsäure eingesetzt werden könnte, findet sich in US 2,585,979 nicht.

Das Verfahren zeichnet sich jedoch bei Verwendung von Trialkylsilylestern der Alkyl- oder Arylsulfon- oder -carbonsäuren besonders aus, da diese Verbindungen wenig toxisch sind.
Die Trialkylsilylester der Alkyl- oder Arylsulfonsäuren sind bevorzugt zur Herstellung der Onium-Salze mit Alkyl- oder Arylsulfonat-Anionen nach dem erfindungsgemäßen Verfahren geeignet.

Die Anhydride der Alkyl- oder Arylcarbonsäuren sind bevorzugt zur Herstellung der Onium-Salze mit Alkyl- oder Arylcarboxylat-Anionen nach dem erfindungsgemäßen Verfahren geeignet.

Geeignete Onium-Halogenide sind Ammoniumhalogenide, Phosphoniumhalogenide, Thiouroniumhalogenide, Guanidiniumhalogenide oder Halogenide mit heterocyclischem Kation, wobei die Halogenide aus der Gruppe Chloride, Bromide oder Iodide ausgewählt werden können. Bevorzugt werden im erfindungsgemäßen Verfahren Chloride oder Bromide eingesetzt. Bevorzugt werden für die Synthese der Thiouronium-Salze Iodide eingesetzt.

Die Onium-Halogenide sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart, Richard C. Larock, Comprehensive Organic Transformations, 2nd Edition, Wiley-VCH, New York, 1999 oder P. Wasserscheid, T. Welton (Eds), Ionic Liquids in Synthesis, Wiley-VCH, 2003 bekannt sind. Dabei kann man auch von an sich bekannten, hier nicht näher erwähnten Varianten Gebrauch machen.

Ammoniumhalogenide können beispielsweise durch die Formel (1)

$[NR_4]^+ Hal^-$ (1),

Phosphoniumhalogenide können beispielsweise durch die Formel (2)

$[PR_4]^+ Hal^-$ (2),

beschrieben werden,
wobei
Hal Cl, Br oder I und
R jeweils unabhängig voneinander
H, wobei nicht alle Substituenten R gleichzeitig H sein dürfen, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann bedeutet,
wobei ein oder mehrere R teilweise oder vollständig mit -F substituiert sein können, wobei jedoch nicht alle vier oder drei R vollständig mit F substituiert sein dürfen,
und wobei ein oder zwei nicht benachbarte und nicht α- oder ω-ständige Kohlenstoffatome des R, durch Atome und/ oder Atomgruppierungen ausgewählt aus der Gruppe -O-, -S-, -S(O)- oder $-SO_2-$ ersetzt sein können.

Ausgeschlossen sind jedoch Verbindungen der Formeln (1) und (2), in denen alle vier oder drei Substituenten R vollständig mit Halogenen substituiert sind, beispielsweise Tris(trifluormethyl)methylammoniumchlorid, Tetra(trifluormethyl)ammoniumchlorid oder Tetra(nonafluorbutyl)ammoniumchlorid.

Thiouroniumhalogenide können beispielsweise durch die Formel (3)

$$[(R^1R^2N)\text{-}C(=SR^7)(NR^3R^4)]^+ \ Hal^- \qquad (3)$$

und Guanidiniumhalogenide können beispielsweise durch die Formel (4)

$$[C(NR^1R^2)(NR^3R^4)(NR^5R^6)]^+ \ Hal^- \qquad (4),$$

beschrieben werden,
wobei
Hal Cl, Br oder I und
$R^1$ bis $R^7$ jeweils unabhängig voneinander
Wasserstoff oder CN, wobei Wasserstoff für $R^7$ ausgeschlossen wird, geradkettiges oder verzweigtes Alkyl mit 1 bis 20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten $R^1$ bis $R^7$ teilweise oder vollständig -F substituiert sein können, wobei jedoch nicht alle Substituenten an einem N-Atom vollständig mit F substituiert sein dürfen,
wobei die Substituenten $R^1$ bis $R^7$ paarweise durch Einfach- oder Doppelbindung miteinander verbunden sein können und wobei ein oder zwei nicht benachbarte, nicht direkt am Heteroatom gebundene und nicht ω-ständige Kohlenstoffatome der Substituenten $R^1$ bis $R^7$ durch Atome und/oder Atomgruppierungen ausgewählt aus der Gruppe -O-, -S-, -S(O)- oder $-SO_2-$ ersetzt sein können.

Halogenide mit heterocyclischem Kation können beispielsweise durch die Formel (5)

$$[HetN]^+ \ Hal^- \qquad (5)$$

beschrieben werden, wobei
Hal Cl, Br oder I und
$HetN^+$ ein heterocyclisches Kation, ausgewählt aus der Gruppe

Imidazolium
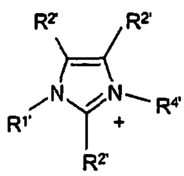
,
1H-Pyrazolium
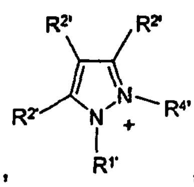
,
3H-Pyrazolium
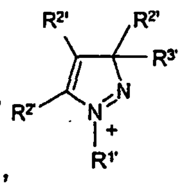
,
4H-Pyrazolium
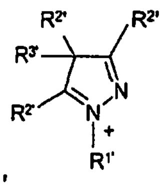
,
1-Pyrazolinium
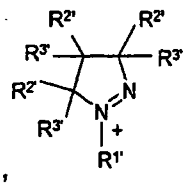
,

2-Pyrazolinium
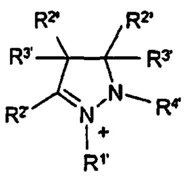
,
3-Pyrazolinium
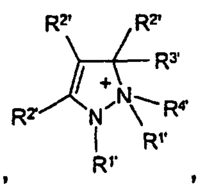
,
2,3-Dihydro-Imidazolinium
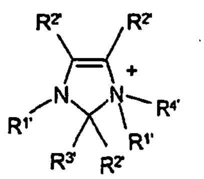
,
4,5-Dihydro-Imidazolinium
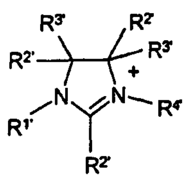
,

2,5-Dihydro-Imidazolinium

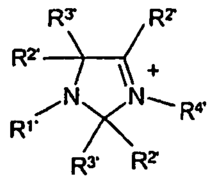

,

Pyrrolidinium

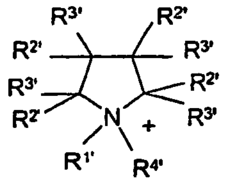

,

1,2,4-Triazolium

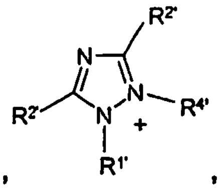

,

1,2,4-Triazolium

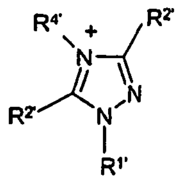

,

1,2,3-Triazolium

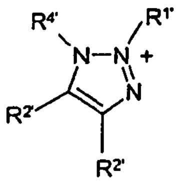

,

1,2,3-Triazolium

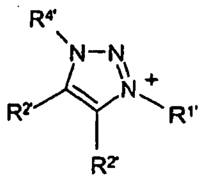

,

Pyridinium

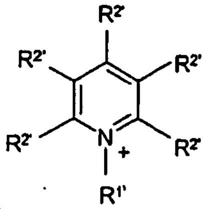

,

Pyridazinium

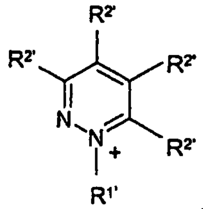

,

Pyrimidinium

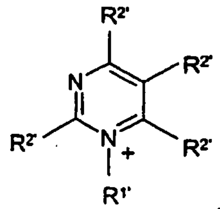

,

Piperidinium

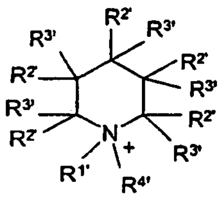

,

Morpholinium

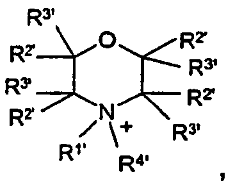

,

Pyrazinium

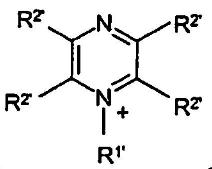

,

Thiazolium

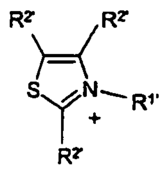

oder

Oxazolium

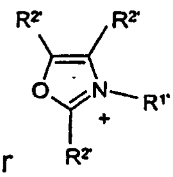

bedeutet, wobei die Substituenten
$R^{1'}$ bis $R^{4'}$ jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7 C-Atomen, das mit Alkylgruppen mit 1-6 C-Atomen substituiert sein kann oder Aryl-$C_1$-$C_6$-alkyl bedeutet,
wobei die Substituenten $R^{1'}$ und $R^{4'}$ teilweise oder vollständig mit F substituiert sein können, wobei jedoch nicht gleichzeitig $R^{1'}$ und $R^{4'}$ CN sind oder vollständig mit F substituiert sein dürfen,
wobei die Substituenten $R^{2'}$ und $R^{3'}$ teilweise oder vollständig mit Halogenen oder teilweise mit $NO_2$ oder CN substituiert sein dürfen und wobei ein oder zwei nicht benachbarte, nicht direkt am Heteroatom gebundene und nicht ω-ständige Kohlenstoffatome der Substituenten $R^{1'}$ bis $R^{4'}$, durch Atome und/oder Atomgruppierungen ausgewählt aus der Gruppe -O-, -S-, -S(O)- oder $-SO_2-$ ersetzt sein können.

Unter vollständig ungesättigten Substituenten werden im Sinne der vorliegenden Erfindung auch aromatische Substituenten verstanden.

Als Substituenten R und $R^1$ bis $R^7$ der Verbindungen der Formeln (1) bis (4) kommen erfindungsgemäß dabei neben Wasserstoff bevorzugt in Frage: $C_1$- bis $C_{20}$-, insbesondere $C_1$- bis $C_{14}$-Alkylgruppen, und gesättigte oder ungesättigte, d.h. auch aromatische, $C_3$- bis $C_7$-Cycloalkylgruppen, die mit $C_1$- bis $C_6$-Alkylgruppen substituiert sein können, insbesondere Phenyl. Die Substituenten R und $R^1$ bis $R^7$ können jedoch ebenfalls durch weitere funktionelle Gruppen substituiert sein, beispielsweise durch CN, $SO_2R'$, $SO_2OR'$ oder COOR'. R' bedeutet nicht oder teilweise fluoriertes $C_1$- bis $C_6$ Alkyl, $C_3$- bis $C_7$-Cycloalkyl, unsubstituiertes oder substituiertes Phenyl.

Die Substituenten R in den Verbindungen der Formel (1) oder (2) können dabei gleich oder verschieden sein. Bevorzugt sind drei Substituenten in den Formeln (1) und (2) gleich und ein Substituent verschieden.

Der Substituent R ist insbesondere bevorzugt Methyl, Ethyl, Isopropyl, Propyl, Butyl, sek.-Butyl, Pentyl, Hexyl, Octyl, Decyl oder Tetradecyl.

Bis zu vier Substituenten des Guanidinium-Kations $[C(NR^1R^2)(NR^3R^4)(NR^5R^6)]^+$ können auch paarweise derart verbunden sein, dass mono-, bi- oder polycyclische Kationen entstehen.

Ohne Einschränkung der Allgemeinheit sind Beispiele für solche Guanidinium-Kationen:

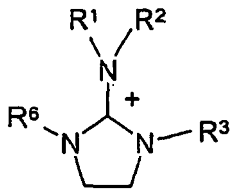 , 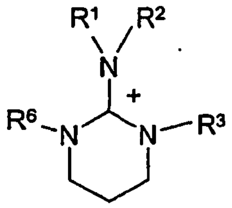 , 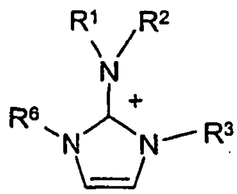 , 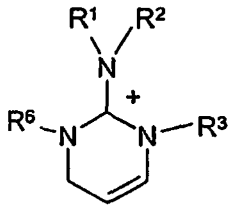 ,

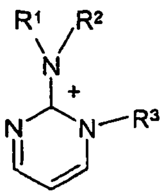 , 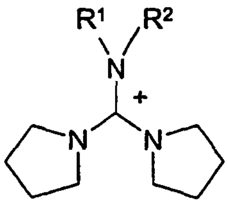 oder 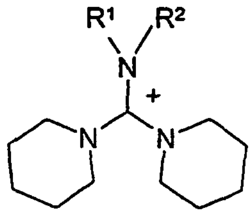 ,

wobei die $R^1$ bis $R^3$ und $R^6$ eine zuvor angegebene oder besonders bevorzugte Bedeutung haben können. Gegebenenfalls können die Carbocyclen oder Heterocyclen der zuvor angegebenen Guanidinium-Kationen noch durch $C_1$- bis $C_6$-Alkyl, $C_1$- bis $C_6$-Alkenyl, $NO_2$, F, Cl, Br, I, $C_1$-$C_6$-Alkoxy, $SCF_3$, $SO_2CF_3$, $SO_2CH_3$, CN, COOR'', $SO_2NR''_2$, $SO_2X'$ oder $SO_3R''$ substituiert sein, wobei X' F, Cl oder Br und R'' ein nicht oder teilweise fluoriertes $C_1$- bis $C_6$-Alkyl oder $C_3$- bis $C_7$-Cycloalkyl wie für R' definiert bedeutet oder durch substituiertes oder unsubstituiertes Phenyl substituiert sein.

Bis zu vier Substituenten des Thiouroniumkations $[(R^1R^2N)-C(=SR^7)(NR^3R^4)]^+$ können auch paarweise derart verbunden sein, dass mono-, bi- oder polycyclische Kationen entstehen.

Ohne Einschränkung der Allgemeinheit sind Beispiele für solche Kationen im folgenden angegeben:

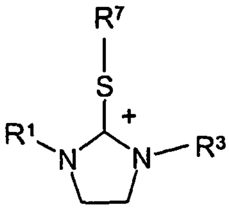 , 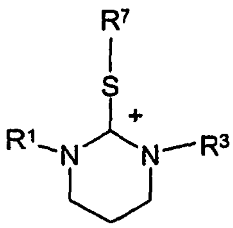 , 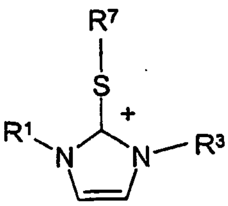 , 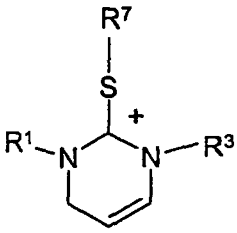 ,

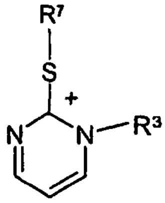 , 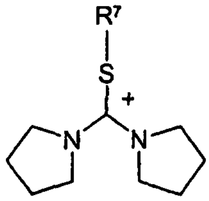 oder 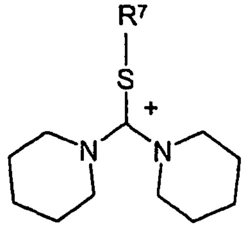 ,

wobei die Substituenten $R^1$, $R^3$ und $R^7$ eine zuvor angegebene oder besonders bevorzugte Bedeutung haben können.

Gegebenenfalls können die Carbocyclen oder Heterocyclen der zuvor angegebenen Thiouronium-Kationen noch durch $C_1$- bis $C_6$-Alkyl, $C_1$- bis $C_6$-Alkenyl, $NO_2$, F, Cl, Br, I, $C_1$-$C_6$-Alkoxy, $SCF_3$, $SO_2CF_3$, $SO_2CH_3$, CN, COOR", $SO_2NR"_2$, $SO_2X'$ oder $SO_3R"$ substituiert sein, wobei X' F, Cl oder Br und R" ein nicht oder teilweise fluoriertes $C_1$- bis $C_6$-Alkyl oder $C_3$- bis $C_7$-Cycloalkyl wie für R' definiert bedeutet oder durch substituiertes oder unsubstituiertes Phenyl substituiert sein.

Die $C_1$-$C_{14}$-Alkylgruppe ist beispielsweise Methyl, Ethyl, Isopropyl, Propyl, Butyl oder sek.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl oder Tetradecyl. Gegebenenfalls perfluoriert, beispielsweise als Difluormethyl, Trifluormethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Vinyl, Allyl, 2- oder 3-Butenyl, Isobutenyl, sek.-Butenyl, ferner 4-Pentenyl, iso-Pentenyl, Hexenyl, Heptenyl, Octenyl, $-C_9H_{17}$, $-C_{10}H_{19}$ bis $-C_{20}H_{39}$; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, $-C_9H_{15}$, $-C_{10}H_{17}$ bis $-C_{20}H_{37}$, vorzugsweise Ethinyl, 1- oder 2-Propinyl, 2- oder 3-Butinyl, 4-Pentinyl, 3-Pentinyl oder Hexinyl.

Aryl-$C_1$-$C_6$-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit -F substituiert sein können, besonders bevorzugt Benzyl oder Phenylpropyl. Der Phenylring oder auch die Alkylenkette können jedoch ebenfalls durch weitere funktionelle Gruppen substituiert sein, beispielsweise durch CN, $SO_2R'$, $SO_2OR'$ oder COOR'. R' hat dabei eine zuvor definierte Bedeutung.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit $C_1$- bis $C_6$-Alkylgruppen substituiert sein können, wobei wiederum die Cycloalkylgruppe oder die mit $C_1$- bis $C_6$-Alkylgruppen substituierte Cycloalkylgruppe auch mit F substituiert sein kann. Die Cycloalkylgruppen können jedoch ebenfalls durch weitere funktionelle Gruppen substituiert sein, beispielsweise durch CN, $SO_2R'$, $SO_2OR'$ oder COOR'. R' hat dabei eine zuvor definierte Bedeutung.

In den Substituenten R, $R^1$ bis $R^6$ oder $R^{1'}$ bis $R^{4'}$ können auch ein oder zwei nicht benachbarte und nicht $\alpha$-ständig zum Heteroatom oder $\omega$gebundene Kohlenstoffatome, durch Atome und/oder Atomgruppierungen ausgewählt aus der Gruppe -O-, -S-, -S(O)- oder $-SO_2-$ ersetzt werden.

Ohne Einschränkung der Allgemeinheit sind Beispiele für derart modifizierte Substituenten R, $R^1$ bis $R^6$ und $R^{1'}$ bis $R^{4'}$:- $OCH_3$, $-OCH(CH_3)_2$, $-CH_2OCH_3$, $-CH_2$-$CH_2$-O-$CH_3$, $-C_2H_4OCH(CH_3)_2$, $-C_2H_4SC_2H_5$, $-C_2H_4SCH(CH_3)_2$, -S (O)$CH_3$, $-SO_2CH_3$, $-SO_2C_6H_5$, $-SO_2C_3H_7$, $-SO_2CH(CH_3)_2$, $-SO_2CH_2CF_3$, $-CH_2SO_2CH_3$, -O-$C_4H_8$-O-$C_4H_9$, -CF3, $-C_2F_5$, $-C_3F_7$, $-C_4F_9$, $-CF_2CF_2H$, $-CF_2CHFCF_3$, $-CF_2CH(CF_3)_2$, $-C_2F_4N(C_2F_5)C_2F_5$, $-CHF_2$, $-CH_2CF_3$, $-C_2F_2H_3$, $-C_3FH_6$, $-CH_2C_3F_7$, $-CH_2C(O)OCH_3$, $-CH_2C_6H_5$ oder-C(O)$C_6H_5$.

In R' ist $C_3$- bis $C_7$-Cycloalkyl beispielsweise Cyclopropyl, Cyclobutyl, Cyclopentyl, Cyclohexyl oder Cycloheptyl.

In R' bedeutet substituiertes Phenyl, durch $C_1$- bis $C_6$-Alkyl, $C_1$- bis $C_6$-Alkenyl, $NO_2$, F, Cl, Br, I, $C_1$-$C_6$-Alkoxy, $SCF_3$, $SO_2CF_3$, $SO_2CH_3$, COOR", $SO_2X'$, $SO_2NR"_2$ oder $SO_3R"$ substituiertes Phenyl, wobei X' F, Cl oder Br und R" ein nicht oder teilweise fluoriertes $C_1$- bis $C_6$-Alkyl oder $C_3$- bis $C_7$-Cycloalkyl wie für R' definiert bedeutet, beispielsweise, o-, m- oder p-Methylphenyl, o-, m- oder p-Ethylphenyl, o-, m- oder p-Propylphenyl, o-, m- oder p-Isopropylphenyl, o-, m- oder p-Nitrophenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-Iodphenyl, weiter bevorzugt 2,3-, 2,4-, 2,5-, 2,6-, 3,4- oder 3,5-Dimethylphenyl, 2,3-, 2,4-, 2,5, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

Die Substituenten $R^1$ bis $R^7$ sind jeweils unabhängig voneinander bevorzugt eine geradkettige oder verzweigte Alkylgruppe mit 1 bis 10 C-Atomen. Die Substituenten $R^1$ und $R^2$, $R^3$ und $R^4$ und $R^5$ und $R^6$ in Verbindungen der Formeln (3) und (4) können dabei gleich oder verschieden sein.

Besonders bevorzugt sind $R^1$ bis $R^7$ jeweils unabhängig voneinander Methyl, Ethyl, n-Propyl, Isopropyl, n-Butyl, sek.-Butyl, Phenyl oder Cyclohexyl, ganz besonders bevorzugt Methyl, Ethyl, n-Propyl, Isopropyl oder n-Butyl.

Als Substituenten $R^{1'}$ bis $R^{4'}$ Von Verbindungen der Formel (5) kommen erfindungsgemäß dabei neben Wasserstoff bevorzugt in Frage: CN, $C_1$- bis $C_{20}$-, insbesondere $C_1$- bis $C_{12}$-Alkylgruppen, und gesättigte oder ungesättigte, d.h. auch aromatische, $C_3$- bis $C_7$-Cycloalkylgruppen, die mit $C_1$- bis $C_6$-Alkylgruppen substituiert sein können, insbesondere Phenyl oder Aryl-$C_l$-$C_6$-alkyl.

Die Substituenten $R^{1'}$ bis $R^{4'}$ können jedoch ebenfalls durch weitere funktionelle Gruppen substituiert sein, beispielsweise

durch CN, $SO_2R'$, $SO_2OR'$ oder COOR'. R' bedeutet nicht oder teilweise fluoriertes $C_1$- bis $C_6$ Alkyl, $C_3$- bis $C_7$-Cycloalkyl, unsubstituiertes oder substituiertes Phenyl.

Die Substituenten $R^{1'}$ und $R^{4'}$ sind jeweils unabhängig voneinander insbesondere bevorzugt CN, Methyl, Ethyl, Isopropyl, Propyl, Butyl, sek.-Butyl, Pentyl, Hexyl, Octyl, Decyl, Cyclohexyl, Phenyl, Phenylpropyl oder Benzyl. Sie sind ganz besonders bevorzugt Methyl, CN, Ethyl, n-Butyl oder Hexyl. In Pyrrolidinium- oder Piperidinium-Verbindungen sind die beiden Substituenten $R^{1'}$ und $R^{4'}$ bevorzugt unterschiedlich.

Der Substituent $R^{2'}$ oder $R^{3'}$ ist jeweils unabhängig voneinander insbesondere Wasserstoff, Dimethylamino, Diethylamino, Methyl, Ethyl, Isopropyl, Propyl, Butyl, sek.-Butyl, Cyclohexyl, Phenyl oder Benzoyl. Besonders bevorzugt ist $R^{2'}$ Wasserstoff, Methyl, Ethyl, Isopropyl, Propyl, Butyl, sek.-Butyl oder Dimethylamino. Ganz besonders bevorzugt sind $R^{2'}$ und $R^{3'}$ Wasserstoff.

Bevorzugt sind die Alkylgruppen als Substituenten R und $R^1$ bis $R^6$ sowie $R^{1'}$ und $R^{4'}$ der heterocyclischen Kationen der Formel (5) unterschiedlich von der Alkylgruppe des eingesetzten Alkylesters der Alkyl- oder Arylsulfonsäure oder der Alkyl- oder Arylcarbonsäure.

Das erfindungsgemäß hergestellte Onium-Sulfonat oder -Carboxylat kann jedoch auch Alkylgruppen im Kation haben, die zu der Alkylgruppe im Ester gleich sind, die jedoch erfindungsgemäß nicht durch Alkylierung eingeführt wurden. Der Focus liegt dann auf der einfachen Reaktionsführung und des besonders niedrigen Halogenid-Gehalts im Endprodukt, wie es für die erfindungsgemäße Reaktion mit Trialkylsilylestern der Alkyl- oder Arylsulfonsäuren oder Alkyl- oder Arylcarbonsäuren oder deren Anhydride von Bedeutung ist.

$HetN^+$ der Formel (5) ist bevorzugt

Imidazolium

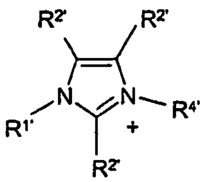

,

Pyrazolium

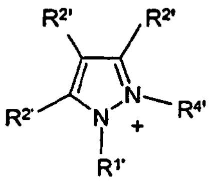

,

Pyrrolidinium

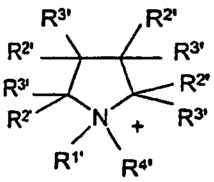

,

Pyridinium

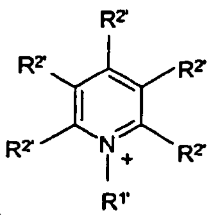

,

Pyrimidinium

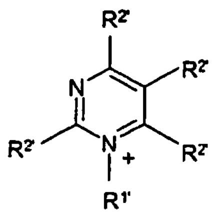

,

oder

Piperidinium

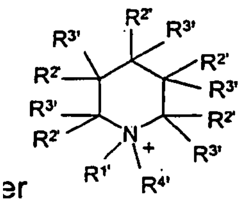

,

wobei die Substituenten $R^{1'}$ bis $R^{4'}$ jeweils unabhängig voneinander eine zuvor beschriebene Bedeutung haben.

$HetN^+$ ist besonders bevorzugt Imidazolium, Pyrrolidinium oder Pyridinium, wie zuvor definiert, wobei die Substituenten $R^{1'}$ bis $R^{4'}$ jeweils unabhängig voneinander eine zuvor beschriebene Bedeutung haben.

Als Alkylester einer Alkyl- oder Arylsulfonsäure wird bevorzugt ein Alkylester mit einer geradkettigen oder verzweigten Alkylgruppe mit 1-8 C-Atomen, bevorzugt mit 1-4 C-Atomen eingesetzt, wobei die Alkylgruppe auch Cl- oder F-Atome enthalten kann. Beispiele für Alkylester von Sulfonsäuren sind beispielsweise Methyltrifluormethansulfonat, Ethyltrifluormethansulfonat, Propyltrifluormethansulfonat, Butyltrifluormethansulfonat, Methylmethansulfonat, Ethylmethansulfonat, Propylmethansulfonat, Butylmethansulfonat, Methylethansulfonat, Ethylethansulfonat, Propylethansulfonat, Butylethansulfonat, Methylbenzolsulfonat, Ethylbenzolsulfonat, Methyl-p-toluensulfonat, Ethyl-p-toluensulfonat, Propyl-p-toluensulfonat, Methyl-p-nitrobenzolsulfonat oder Ethyl-p-nitroenzolsulfonat.

Besonders bevorzugt werden Methyltrifluormethansulfonat, Ethyltrifluormethansulfonat, Methylmethansulfonat, Methylbenzolsulfonat, Methyl-p-toluensulfonat oder Ethyl-p-toluensulfonat eingesetzt. Ganz besonders bevorzugt wird Methyltrifluormethansulfonat oder Ethyltrifluormethansulfonat eingesetzt.

Alkylester oder Trialkylsilylester der Chlorsulfonsäure sind auch im erfindungsgemäßen Verfahren verwendbar, beispielsweise Methylchlorsulfonat oder Trimethylsilylchlorsulfonat.

Als Trialkylsilylester einer Alkyl- oder Arylsulfonsäure wird bevorzugt ein Trialkylsilylester mit einer geradkettigen oder verzweigten Alkylgruppe mit 1-8 C-Atomen, bevorzugt mit 1-4 C-Atomen eingesetzt, wobei jede Alkylgruppe unabhängig voneinander ist. Bevorzugt sind die Alkylgruppen am Silicium gleich. Trialkylsilylester von Sulfonsäuren sind beispielsweise $CF_3$-$SO_2$-O-Si$(CH_3)_3$, $CF_3$-$SO_2$-O-Si$(CH_3)_2$(t-$C_4H_9$), $CF_3$-$SO_2$-O-Si$(C_2H_5)_2$(i-$C_3H_7$), $CF_3$-$SO_2$-O-Si$(C_2H_5)_3$, $CF_3$-$SO_2$-O-Si(i-$C_3H_7)_3$, $CF_3$-$SO_2$-O-Si-(n-$C_4H_9)_3$, $CH_3SO_2$-O-Si$(CH_3)_3$, $CH_3$-$SO_2$-O-Si$(CH_3)_2$(n-$C_4H_9$), ($C_2H_5$)-$SO_2$-O-Si-$(CH_3)_3$, ($C_2H_5$)-$SO_2$-O-Si$(C_2H_5)_3$, $C_2F_5$-$SO_2$-O-Si$(CH_3)_3$, $C_4F_9SO_2$-O-Si$(CH_3)_3$, $C_4F_9$-$SO_2$-O-Si$(C_2H_5)_3$, $C_4F_9$-$SO_2$-O-Si$(CH_3)_2$(n-$C_4H_9$), $C_4F_9$-$SO_2$-O-Si($CH_3$)(n-$C_4H_9)_2$, $C_8F_{17}$-$SO_2$-O-Si$(CH_3)_3$, $C_6F_5$-$SO_2$-O-Si$(CH_3)_3$ oder (p-$CH_3$)$C_6H_4$-$SO_2$-O-Si$(CH_3)_3$. Besonders bevorzugt werden $CF_3$-$SO_2$-O-Si$(CH_3)_3$, $CH_3SO_2$-O-Si$(CH_3)_3$, ($C_2H_5$)-$SO_2$-O-Si$(C_2H_5)_3$, $C_4F_9SO_2$-O-Si$(CH_3)_3$, $C_8F_{17}$-$SO_2$-O-Si$(CH_3)_3$, $C_6F_5$-$SO_2$-O-Si$(CH_3)_3$ oder (p-$CH_3$)$C_6H_4$-$SO_2$-O-Si$(CH_3)_3$, ganz besonders bevorzugt werden Trimethylsilylmethansulfonat ($CH_3SO_2$-O-Si$(CH_3)_3$), Trimethylsilylfluorbenzolsulfonat ($C_6F_5SO_2$-O-Si$(CH_3)_3$), Trimethylsilyltoluensulfonat ((p-$CH_3$)$C_6H_4$-$SO_2$-O-Si$(CH_3)_3$) oder Trimethylsilyltrifluormethansulfonat ($CF_3SO_2$-O-Si$(CH_3)_3$) eingesetzt.

Als Alkylester einer Alkyl- oder Arylcarbonsäure wird bevorzugt ein Alkylester mit einer geradkettigen oder verzweigten Alkylgruppe mit 1-8 C-Atomen, bevorzugt mit 1-4 C-Atomen eingesetzt. Alkylester von Carbonsäuren sind beispielsweise Methyltrifluoracetat, Ethyltrifluoracetat, Propyltrifluoracetat, Butyltrifluoracetat, tert-Butylacetat, tert-Butylpropionat, iso-Propylbenzoat, tert-Butylbenzoat, Methyl-4-nitrobenzoat oder Methylpentafluorbenzoat. Besonders bevorzugt werden Methyltrifluoracetat oder Ethyltrifluoracetat eingesetzt.

Als Trialkylsilylester einer Alkyl- oder Arylcarbonsäure wird bevorzugt ein Trialkylsilylester mit einer geradkettigen oder verzweigten Alkylgruppe mit 1-8 C-Atomen, bevorzugt mit 1-4 C-Atomen eingesetzt, wobei jede Alkylgruppe unabhängig voneinander ist. Bevorzugt sind die Alkylgruppen am Silicium gleich. Trialkylsilylester von Carbonsäuren sind beispielsweise $CF_3$-C(O)-O-Si$(CH_3)_3$, $CF_3$-C(O)-O-Si$(CH_3)_2$(t-$C_4H_9$), $CF_3$-C(O)-O-Si$(C_2H_5)_2$(i-$C_3H_7$), $CF_3$-C(O)-O-Si$(C_2H_5)_3$, $CF_3$-C(O)-O-Si(i-$C_3H_7)_3$, $CF_3$-C(O)-O-Si-(n-$C_4H_9)_3$, $CH_3$-C(O)-O-Si$(CH_3)_3$, $CH_3$-C(O)-O-Si$(CH_3)_2$(n-$C_4H_9$), ($C_2H_5$)-C(O)-O-Si-$(CH_3)_3$, ($C_2H_5$)-C(O)-O-Si$(C_2H_5)_3$, $C_2F_5$-C(O)-O-Si$(CH_3)_3$, $C_4F_9$-C(O)-O-Si$(CH_3)_3$, $C_4F_9$-C(O)-O-Si$(C_2H_5)_3$, $C_4F_9$-C(O)-O-Si$(CH_3)_2$(n-$C_4H_9$), $C_4F_9$-C(O)-O-Si($CH_3$)(n-$C_4H_9)_2$, $C_8F_{17}$-C(O)-O-Si$(CH_3)_3$, $C_6H_5$-C(O)-O-Si$(CH_3)_3$ oder (p-$NO_2$)$C_6H_4$-C(O)-O-Si$(CH_3)_3$. Besonders bevorzugt werden $CF_3$-C(O)-O-Si$(CH_3)_3$, $C_4F_9$-C(O)-O-Si$(CH_3)_3$, $C_8F_{17}$-C(O)-O-Si$(CH_3)_3$, $C_6F_5$-C(O)-O-Si$(CH_3)_3$ oder (p-$NO_2$)$C_6H_4$-C(O)-O-Si$(CH_3)_3$, ganz besonders bevorzugt werden Trimethylsilylpentafluorbenzoat ($C_6F_5$-C(O)-O-Si$(CH_3)_3$) oder Trimethylsilyltrifluoracetat ($CF_3$-C(O)-O-Si$(CH_3)_3$) eingesetzt.

Als Säureanhydrid wird bevorzugt das Anhydrid einer geradkettigen oder verzweigten Alkylcarbonsäure mit 1-8 C-Atomen der Alkylgruppe, bevorzugt mit 1-4 C-Atomen der Alkylgruppe, das Anhydrid einer aromatischen Carbonsäure, das Anhydrid einer Sulfonsäure oder das gemischte Anhydrid einer Carbonsäure und einer Sulfonsäure eingesetzt. Geeignete Anhydride sind Trifluoressigsäureanhydrid, Bernsteinsäureanhydrid, Pentafluorpropansäureanhydrid, Trifluormethansulfonsäureanhydrid oder gemischte Anhydride von Trifluoressigsäure, Essigsäure, Propionsäure, Weinsäure, Malonsäure, Nicotinsäure, Aminoessigsäure oder Benzoesäure. Besonders bevorzugt wird Trifluoressigsäureanhydrid eingesetzt.

Für den Einsatz im erfindungsgemäßen Verfahren sind ebenfalls Alkyl- oder Trialkylsilylester von 2,4,6-Trinitrophenol (Pikrinsäure) oder 2,4-Dinitrophenol geeignet.

Die eingesetzten Ester oder Anhydride sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Ein allgemeines Schema fasst das erfindungsgemäße Verfahren zusammen:

$[NR_4]^+ Hal^-$ (1) oder $[PR_4]^+ Hal^-$ (2) oder

oder $[(R^1R^2N)\text{-}C(=SR^7)(NR^3R^4)]^+ Hal^-$ (3) oder $[C(NR^1R^2)(NR^3R^4)(NR^5R^6)]^+ Hal^-$ (4)

oder $[HetN]^+ Hal^-$ (5)

\+ [Alkylester] oder [Trialkylsilylester]
oder Alkyl-$S(O)_2$-O-$S(O_2)$-Alkyl
oder Alkyl(Aryl)C(O)-O-C(O)Alkyl(Aryl)

$[NR_4]^+$[Säureanion]$^-$ (6) oder $[PR_4]^+$[Säureanion]$^-$ (7) oder

$[(R^1R^2N)\text{-}C(=SR^7)(NR^3R^4)]^+$ [Säureanion]$^-$ (8) oder

$[C(NR^1R^2)(NR^3R^4)(NR^5R^6)]^+$ [Säureanion]$^-$ (9) oder

$[HetN]^+$ [Säureanion]$^-$ (10)

\+ Alkyl-Hal oder
Alkyl(Aryl)C(O)-Hal
oder
Trialkylsilyl-Hal oder
Alkyl-$S(O)_2$-Hal

Die Substituenten R, $R^1$ bis $R^7$ und $HetN^+$ der Verbindungen der Formeln (1) bis (10) entsprechen den Bedeutungen, wie zuvor beschrieben.

Die Reaktion mit Alkylestern von Alkyl- oder Arylsulfonsäuren wird erfindungsgemäß bei Raumtemperatur, d.h. in der Regel bei Temperaturen zwischen 10° und 30°C durchgeführt. Die Reaktion mit Alkylestern von Alkyl- oder Arylcarbonsäuren, Trialkylsilylestern von Alkyl- oder Arylsulfonsäuren oder Alkyl- oder Arylcarbonsäuren findet in der Regel bei Temperaturen zwischen 0° und 50°C, vorzugsweise 10° bis 30°C, besonders bevorzugt bei Raumtemperatur statt. Die Reaktion mit Säureanhydriden erfolgt bei Temperaturen zwischen 20° bis 180°C, bevorzugt zwischen 50° und 100°C. Sie kann jedoch auch bei Raumtemperatur stattfinden.

Es wird kein Lösungsmittel benötigt. Es können jedoch auch Lösungsmittel eingesetzt werden, beispielsweise Dimethoxyethan, Acetonitril, Aceton, Tetrahydrofuran, Dimethylformamid, Dimethylsulfoxid, Dioxan, Propionitril oder Gemische untereinander.

Die Umsetzung wird mit einem Überschuss oder equimolarer Menge an Alkyl- oder Trialkylsilylester einer Alkyl- oder Arylsulfonsäure oder einer Alkyl- oder Arylcarbonsäure oder dem entsprechenden Anhydrid durchgeführt.

Die beschriebene Methode ist auch für die Reinigung der Onium-Salze geeignet. Das bedeutet, dass der durch Halogenid-Ionen verunreinigten ionischen Flüssigkeit, beispielsweise 1-Butyl-3-methylimidazolium Trifluormethansulfonat, verunreinigt mit 1-Butyl-3-methylimidazoliumchlorid, ein entsprechender erfindungsgemäßer Ester oder ein Anhydrid der Säure des Anions, beispielsweise mit Trimethylsilyltrifluormethansulfonat zugegeben wird. Die Verunreinigung reagiert ab und man kommt zur halogenidreduzierten ionischen Flüssigkeit.

Besonders geeignet ist die Synthese auch für heterocyclische Trifluormethansulfonate, wobei ein positivierter Stickstoff des Heterocyclus eine CN-Gruppe trägt. Solche Verbindungen sind hervorragende Cyanierungsreagenzien. Der Austausch des bekannten Anions Tetrafluoroborat zu Trifluormethansulfonat führt zu stabileren Verbindungen.

Gegenstand der Erfindung sind daher ebenfalls Triflate der Formel (11)

$$HetN^+ [CF_3SO_3]^- \qquad (11),$$

wobei
$HetN^+$ ein heterocyclisches Kation, ausgewählt aus der Gruppe

Imidazolium
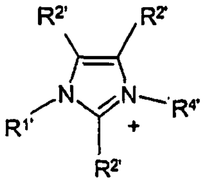
,
1H-Pyrazolium
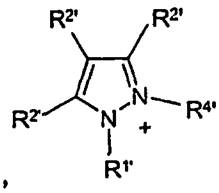
,
3H-Pyrazolium
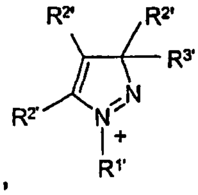
,
4H-Pyrazolium
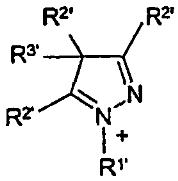
,
1-Pyrazolinium
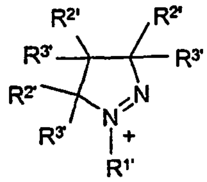
,

2-Pyrazolinium
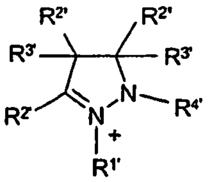
,
3-Pyrazolinium
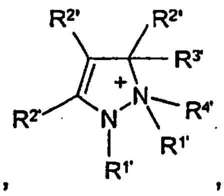
,
2,3-Dihydro-Imidazolinium
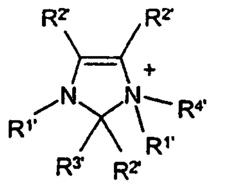
,
4,5-Dihydro-Imidazolinium
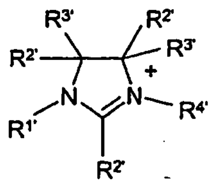
,

2,5-Dihydro-Imidazolinium
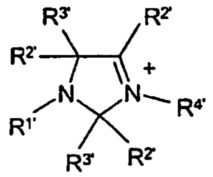
,
Pyrrolidinium
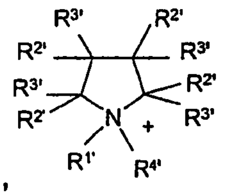
,
1,2,4-Triazolium
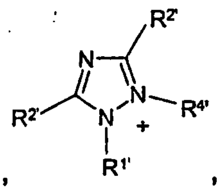
,
1,2,4-Triazolium
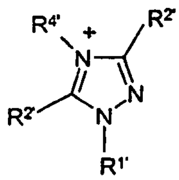
,

1,2,3-Triazolium
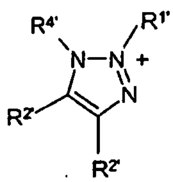
,
1,2,3-Triazolium
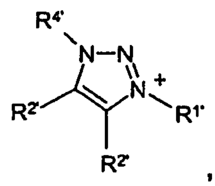
,
Pyridinium
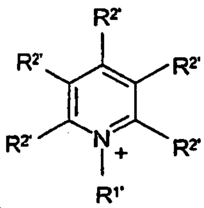
,
Pyridazinium
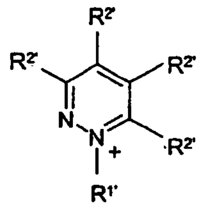
,
Pyrimidinium
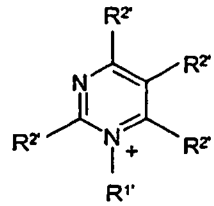
,

Piperidinium
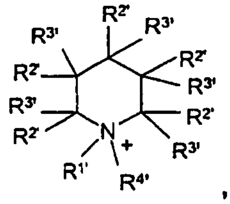
,
Morpholinium
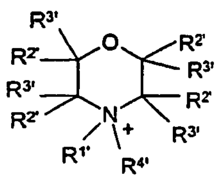
,
Pyrazinium
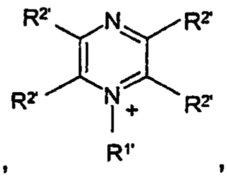
,
Thiazolium
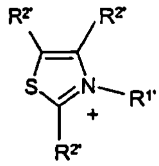
oder
Oxazolium
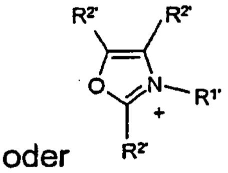

bedeutet, wobei $R^{1'}$ oder $R^{4'}$ CN bedeutet und die übrigen Substituenten $R^{1'}$ bis $R^{4'}$ jeweils unabhängig voneinander Wasserstoff,

geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7 C-Atomen, das mit Alkylgruppen mit 1-6 C-Atomen substituiert sein kann oder Aryl-$C_1$-$C_6$-alkyl bedeutet,
wobei ein oder mehrere Substituenten $R^{2'}$ und $R^{3'}$ teilweise oder vollständig mit -F substituiert sein können,
wobei jedoch nicht gleichzeitig $R^{1'}$ und $R^{4'}$ CN sind
und wobei ein oder zwei nicht benachbarte, nicht direkt am Heteroatom gebundene und nicht ω-ständige Kohlenstoffatome der Substituenten $R^{1'}$ bis $R^{4'}$, durch Atome und/oder Atomgruppierungen ausgewählt aus der Gruppe -O-, -S-, -S(O)- oder -$SO_2$- ersetzt sein können.

Als Substituenten $R^{1'}$ bis $R^{4'}$ von Verbindungen der Formel (5) kommen erfindungsgemäß dabei neben Wasserstoff bevorzugt in Frage: CN, $C_1$- bis $C_{20}$-, insbesondere $C_1$- bis $C_{12}$-Alkylgruppen, und gesättigte oder ungesättigte, d.h. auch aromatische, $C_3$- bis $C_7$-Cycloalkylgruppen, die mit $C_1$- bis $C_6$-Alkylgruppen substituiert sein können, insbesondere Phenyl oder Aryl-$C_1$-$C_6$-alkyl.

Ein Substituent $R^{1'}$ oder $R^{4'}$ ist definitionsgemäß CN. Der zweite Substituent $R^{1'}$ oder $R^{4'}$ ist insbesondere bevorzugt Methyl, Ethyl, Isopropyl, Propyl, Butyl, sek.-Butyl, Pentyl, Hexyl, Octyl, Decyl, Cyclohexyl, Phenyl, Phenylpropyl oder Benzyl, ganz besonders bevorzugt Methyl, Ethyl, n-Butyl oder Hexyl.

Der Substituent $R^{2'}$ oder $R^{3'}$ ist jeweils unabhängig voneinander insbesondere Wasserstoff, Dimethylamino, Diethylamino, Methyl, Ethyl, Isopropyl, Propyl, Butyl, sek.-Butyl, Cyclohexyl, Phenyl oder Benzyl. Besonders bevorzugt ist $R^{2'}$ Wasserstoff, Methyl, Ethyl, Isopropyl, Propyl, Butyl, sek.-Butyl oder Dimethylamino. Ganz besonders bevorzugt sind $R^{2'}$ und $R^{3'}$ jeweils unabhängig voneinander Wasserstoff.

$HetN^+$ der Formel (11) ist bevorzugt

Imidazolium

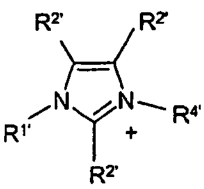

,

Pyrazolium

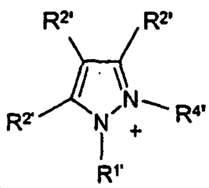

,

Pyrrolidinium

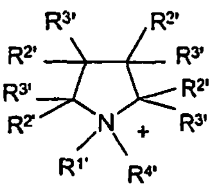

,

Pyridinium

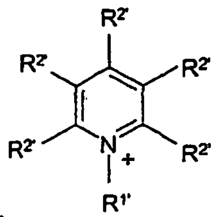

,

Pyrimidinium

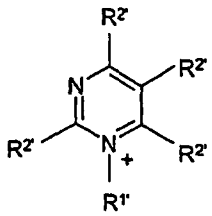

oder

Piperidinium

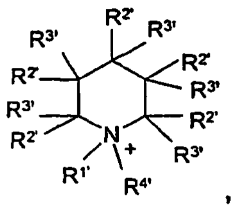

,

wobei die Substituenten $R^{1'}$ bis $R^{4'}$ jeweils unabhängig voneinander eine zuvor beschriebene Bedeutung haben.

$HetN^+$ ist besonders bevorzugt Imidazolium, Pyrrolidinium oder Pyridinium, wie zuvor definiert, wobei die Substituenten $R^{1'}$ bis $R^{4'}$ jeweils unabhängig voneinander eine zuvor beschriebene Bedeutung haben, ganz besonders bevorzugt Pyridinium.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.
Es versteht sich für den Fachmann von selbst, dass in den vorab und nachfolgend genannten Verbindungen Substituenten wie beispielsweise H, N, O, Cl, F durch die entsprechenden Isotope ersetzt sein können.

Die NMR-Spektren wurden an Lösungen in deuterierten Lösungsmitteln bei 20°C an einem Bruker ARX 400 Spektrometer mit einem 5 mm Breitbandkopf $^{1}H$/BB mit Deuterium Lock gemessen, falls nicht in den Beispielen angegeben. Die Messfrequenzen der verschiedenen Kerne sind:
$^{1}H$: 400,13 MHz und $^{19}F$: 276,50 MHz. Die Methode der Referenzierung wird bei jedem Spektrum bzw. bei jedem Datensatz separat angegeben.

Beispiel 1:

Synthese von 1-Hexyl-3-methylimidazolium Trifluormethansulfonat (Triflat)

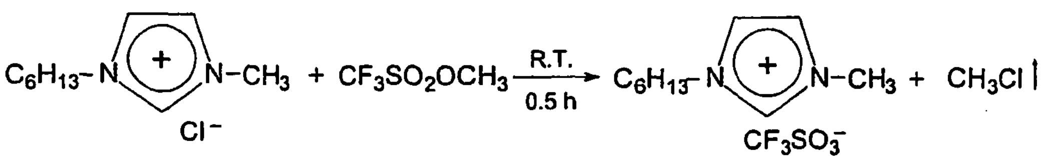


Eine Mischung von 1.80 g (8.88 mmol) 1-Hexyl-3-methylimidazoliumchlorid und 1.52 g (9.26 mmol) Methyltriflat, $CF_3SO_2OCH_3$, werden bei Raumtemperatur für 30 Minuten gerührt. NMR-Messungen zeigen die Vollständigkeit der Reaktion. Der Rückstand wird für 30 Minuten im Vakuum bei 13.3 Pa und 120°C (Ölbadtemperatur) getrocknet. Man erhält 2.80 g 1-Hexyl-3-methylimidazolium Triflat in annähernd quantitativer Ausbeute.

$^{1}H$ NMR (Referenz: TMS ; $CD_3CN$), ppm: 0.87 m ($CH_3$); 1.29 m (3$CH_2$), 1.81 m ($CH_2$); 3.82 s ($CH_3$); 4.11 t ($CH_2$); 7.35 d,d (CH) ; 7.39 d,d (CH); 8.55 br. s. (CH); $^{3}J_{H,H}$ = 6.9 Hz ; $J_{H,H}$ = 1.5 Hz.
$^{19}F$ NMR (Referenz: $CCl_3F$ - intern; $CD_3CN$), ppm: -78.1 s ($CF_3$).

Beispiel 2:

Synthese von 1-Butyl-3-methylimidazolium Triflat

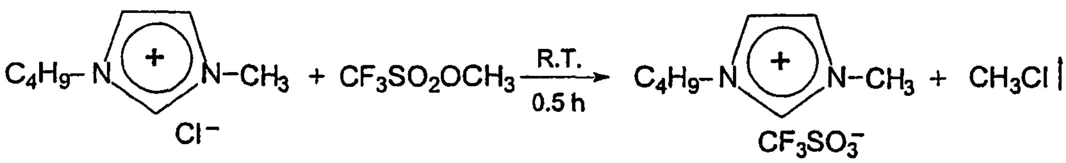


Analog zu Beispiel 1 werden 1.04 g (5.95 mmol) 1-Butyl-3-methylimidazoliumchlorid mit 1.67 g (10.2 mmol) Methyltriflat umgesetzt. Man erhält 1.71 g 1-Butyl-3-methylimidazolium Triflat in annähernd quantitativer Ausbeute.

$^{1}H$ NMR (Referenz: TMS; $CD_3CN$), ppm: 0.92 t ($CH_3$); 1.31 m ($CH_2$); 1.80 m ($CH_2$); 3.82 s ($CH_3$); 4.12 t ($CH_2$); 7.34 d,d (CH); 7.38 d,d (CH); 8.51 br. s. (CH); $^{3}J_{H,H}$ = 7.4 Hz ; $^{3}J_{H,H}$ = 7.1 Hz; $J_{H,H}$ = 1.7 Hz.
$^{19}F$ NMR (Referenz: $CCl_3F$ - intern; $CD_3CN$), ppm: -78.11 s ($CF_3$).

Beispiel 3:

Synthese von 1-Ethylpyridinium Triflat

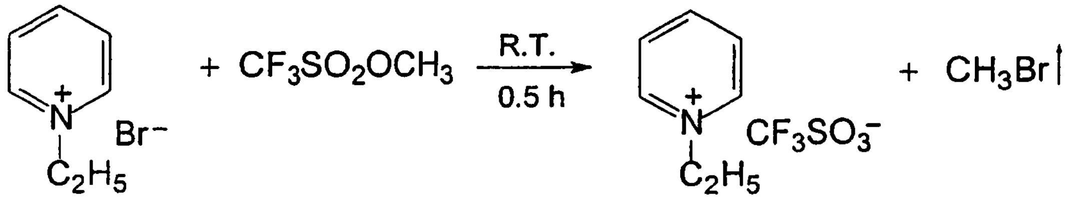


Analog zu Beispiel 1 werden 1.56 g (8.29 mmol) 1-Ethylpyridiniumbromid mit 1.67 g (10.2 mmol) Methyltriflat umgesetzt. Man erhält 2.13 g 1-Ethylpyridinium Triflat in annähernd quantitativer Ausbeute.

$^{1}H$ NMR (Referenz: TMS; $CD_3CN$), ppm: 1.58 t ($CH_3$); 4.59 q ($CH_2$); 8.02 m (2CH); 8.49 t,t (CH); 8.78 d (2CH); $^{3}J_{H,H}$ = 7.3 Hz; $^{3}J_{H,H}$ = 7.6 Hz; $^{3}J_{H,H}$ = 6.1 Hz; $^{4}J_{H,H}$ = 1.2 Hz.
$^{19}F$ NMR (Referenz: $CCl_3F$ - intern; $CD_3CN$), ppm: -78.0 s ($CF_3$).

Beispiel 4:

Synthese von 1-Butylpyridinium Triflat

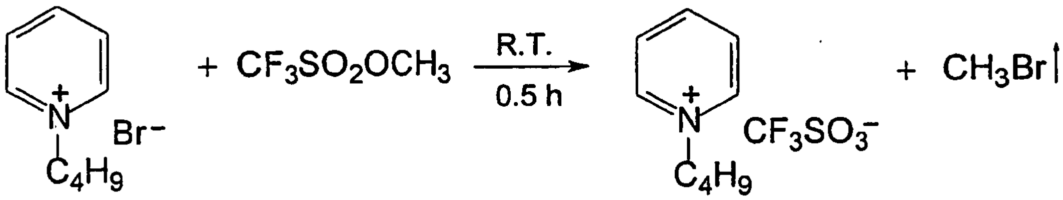


Analog zu Beispiel 1 werden 4.31 g (19.9 mmol) 1-Butylpyridiniumbromid mit 4.41 g (26.9 mmol) Methyltriflat umgesetzt. Man erhält 5.68 g 1-Butylpyridinium Triflat in annähernd quantitativer Ausbeute.

$^{1}H$ NMR (Referenz: TMS; $CD_3CN$), ppm: 0.93 t ($CH_3$); 1.35 m ($CH_2$); 1.93 m ($CH_2$); 4.53 t ($CH_2$); 8.02 m (2CH); 8.50 t (CH); 8.74 d (2CH); $^{3}J_{H,H}$ = 7.4 Hz; $^{3}J_{H,H}$ = 7.6 Hz; $^{3}J_{H,H}$ = 7.9 Hz; $^{3}J_{H,H}$ = 6.1 Hz.
$^{19}F$ NMR (Referenz: $CCl_3F$ - intern; $CD_3CN$), ppm: -78.0 s ($CF_3$).

Beispiel 5:

Synthese von Trihexyl-tetradecyl-phosphonium Triflat

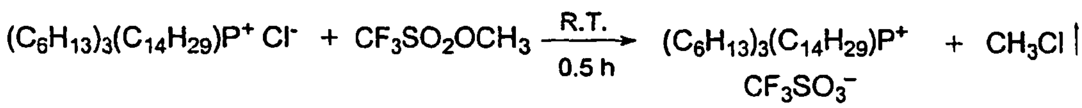


Analog zu Beispiel 1 werden 1.75 g (3.37 mmol) Trihexyl-tetradecylphosphoniumchlorid mit 2.59 g (15.8 mmol) Methyltriflat für 30 Minuten umgesetzt. Man erhält 2.13 g Trihexyl-tetradecyl-phosphonium Triflat in annähernd quantitativer Ausbeute.

$^{1}H$ NMR (Referenz: TMS; $CD_3CN$), ppm: 0.84-0.94 m ($4CH_3$), 1.24-1.37 m ($16CH_2$), 1.37-1.57 m ($8CH_2$), 1.99-2.09 m ($4CH_2$).
$^{19}F$ NMR (Referenz: $CCl_3F$ - intern; $CD_3CN$), ppm: -78.0 s ($CF_3$).
$^{31}P$ {$^{1}H$} NMR (Referenz: 85% $H_3PO_4$ - extern; $CD_3CN$), ppm: 33.5.

Beispiel 6:

Synthese von 1-Ethylpyridinium Triflat

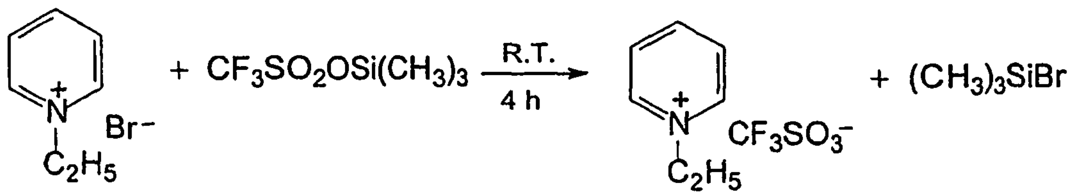


Eine Mischung von 1.85 g (9.84 mmol) 1-Ethylpyridiniumbromid und 2.54 g (11.4 mmol) Trimethylsilyltriflat, $CF_3SO_2OSi(CH_3)_3$, werden bei Raumtemperatur für 4 Stunden gerührt. NMR-Messungen zeigen die Vollständigkeit der Reaktion. Der Rückstand wird für 30 Minuten im Vakuum bei 13.3 Pa und 120°C (Ölbadtemperatur) getrocknet. Man erhält 2.53 g 1-Ethylpyridinium Triflat in annähernd quantitativer Ausbeute.

Die NMR-Spektren entsprechen denen von Beispiel 3.

Beispiel 7:

Synthese von 1-Butylpyridinium Triflat

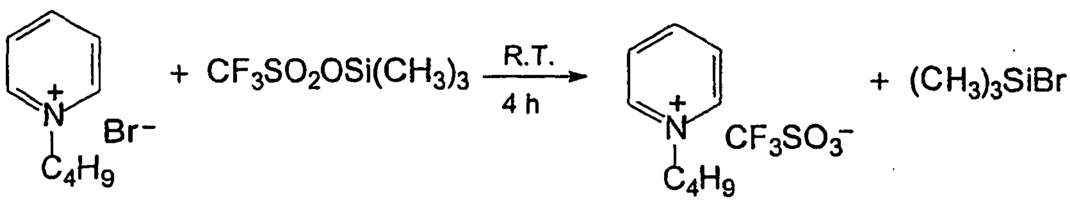


Analog zu Beispiel 6 werden 1.42 g (6.57 mmol) 1-Butylpyridiniumbromid mit 1.73 g (7.78 mmol) Trimethylsilyltriflat umgesetzt. Man erhält 1.87 g 1-Butylpyridinium Triflat in annähernd quantitativer Ausbeute.

Die NMR-Spektren entsprechen denen von Beispiel 4.

Beispiel 8:

Synthese von 1-Butyl-3-methylimidazolium Triflat

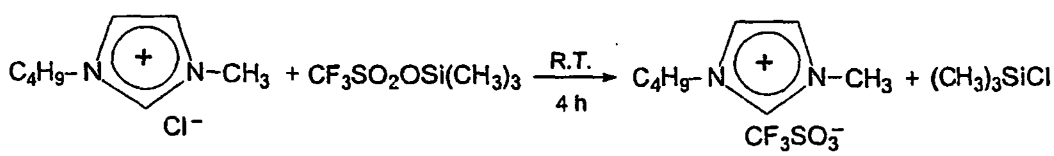


Analog zu Beispiel 6 werden 1.67 g (9.56 mmol) 1-Butyl-3-methylimidazoliumchlorid mit 2.36 g (10.6 mmol) Trimethylsilyltriflat umgesetzt. Man erhält 2.75 g 1-Butyl-3-methylimidazolium Triflat in annähernd quantitativer Ausbeute.

Die NMR-Spektren entsprechen denen von Beispiel 2.

Beispiel 9:

Synthese von 1-Hexyl-3-methylimidazolium Triflat

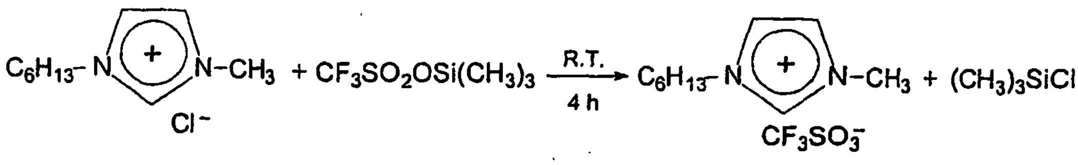


Analog zu Beispiel 6 werden 1.81 g (8.93 mmol) 1-Hexyl-3-methylimidazoliumchlorid mit 2.62 g (11.79 mmol) Trimethylsilyltriflat umgesetzt. Man erhält 2.81 g 1-Hexyl-3-methylimidazolium Triflat in annähernd quantitativer Ausbeute.

Die NMR-Spektren entsprechen denen von Beispiel 1.

Beispiel 10:

Synthese von Trihexyl-tetradecyl-phosphonium Triflat

$$(C_6H_{13})_3(C_{14}H_{29})P^+\,Cl^- + CF_3SO_2OSi(CH_3)_3 \xrightarrow[4\,h]{R.T.} (C_6H_{13})_3(C_{14}H_{29})P^+\;CF_3SO_3^- + (CH_3)_3SiCl$$

Analog zu Beispiel 6 werden 1.64 g (3.16 mmol) Trihexyl-tetradecylphosphoniumchlorid mit 0.91 g (4.09 mmol) Trimethylsilyltriflat umgesetzt. Man erhält 2.00 g Trihexyl-tetradecyl-phosphonium Triflat in annähernd quantitativer Ausbeute.

Die NMR-Spektren entsprechen denen von Beispiel 5.

Beispiel 11:

Analog zu Beispiel 6 werden
1-Methylimidazoliumchlorid mit Trimethylsilyltriflat zu 1-Methylimidazolium Triflat;
1-Butylimidazoliumchlorid mit Trimethylsilyltriflat zu 1-Butylimidazolium Triflat;
1,3-Dimethylimidazoliumchlorid mit Trimethylsilyltriflat zu 1,3-Dimethylimidazolium Triflat;
1-Ethyl-3-methylimidazoliumchlorid mit Trimethylsilyltriflat zu 1-Ethyl-3-methylimidazolium Triflat;
1-Butyl-3-ethylimidazoliumchlorid mit Trimethylsilyltriflat zu 1-Butyl-3-ethylimidazolium Triflat;
1-Methyl-3-pentylimidazoliumchlorid mit Trimethylsilyltriflat zu 1-Methyl-3-pentylimidazolium Triflat;
1-Octyl-3-methylimidazoliumchlorid mit Trimethylsilyltriflat zu 1-Octyl-3-methylimidazolium Triflat;
1-Benzyl-3-methylimidazoliumchlorid mit Trimethylsilyltriflat zu 1-Benzyl-3-methylimidazolium Triflat;
1-Phenylpropyl-3-methylimidazoliumchlorid mit Trimethylsilyltriflat zu 1-Phenylpropyl-3-methylimidazolium Triflat;
1-Ethyl-2,3-dimethylimidazoliumchlorid mit Trimethylsilyltriflat zu 1-Ethyl-2,3-dimethylimidazolium Triflat;
1-Butyl-2,3-dimethylimidazoliumchlorid mit Trimethylsilyltriflat zu 1-Butyl-2,3-dimethylimidazolium Triflat;
1-Hexyl-2,3-dimethylimidazoliumchlorid mit Trimethylsilyltriflat zu 1-Hexyl-2,3-dimethylimidazolium Triflat;
1-Hexylpyridiniumbromid mit Trimethylsilyltriflat zu 1-Hexylpyridinium Triflat;
1-Butyl-3-methylpyridiniumbromid mit Trimethylsilyltriflat zu 1-Butyl-3-methylpyridinium Triflat;
1-Butyl-3-ethylpyridiniumbromid mit Trimethylsilyltriflat zu 1-Butyl-3-ethylpyridinium Triflat oder
Methyltrioctylammoniumchlorid mit Trimethylsilyltriflat zu Methyltrioctylammonium Triflat umgesetzt.

Beispiel 12:

Synthese von 1-Butyl-3-methylimidazolium Methylsulfonat

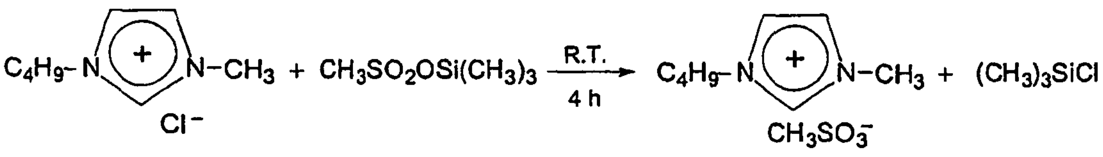


Analog zu Beispiel 6 werden 2.06 g (11.8 mmol) 1-Butyl-3-methylimidazoliumchlorid mit 1.99 g (11.8 mmol) Trimethylsilylmethansulfonat, $CH_3SO_3SI(CH_3)_3$, umgesetzt. Man erhält 2.72 g 1-Butyl-3-methylimidazolium Methylsulfonat in annähernd quantitativer Ausbeute.

$^1H$ NMR (Referenz: TMS; $CD_3CN$), ppm: 0.91 t ($CH_3$); 1.30 m ($CH_2$); 1.80 m ($CH_2$); 2.47 s ($CH_3$); 3.87 s ($CH_3$); 4.18 t ($CH_2$); 7.48 d,d (CH); 7.51 d,d (CH); 9.23 br. s. (CH); $^3J_{H,H}$ = 7.4 Hz; $^3J_{H,H}$ = 7.2 Hz; $J_{H,H}$ = 1.7 Hz.

Beispiel 13:

Synthese von 1-Butyl-3-methylimidazolium Benzolsulfonat

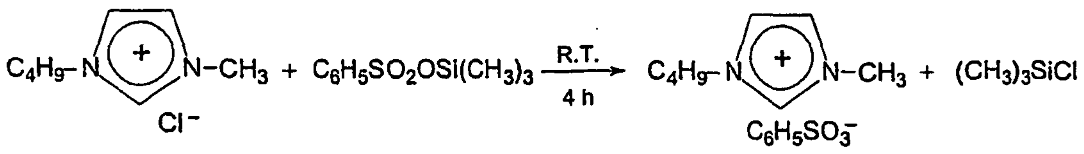


Analog zu Beispiel 6 werden 1.57 g (8.99 mmol) 1-Butyl-3-methylimidazoliumchlorid mit 2.09 g (9.07 mmol) Trimethylsilylbenzolsulfonat, $C_6H_5SO_3Si(CH_3)_3$, umgesetzt. Die Trocknung im Vakuum erfolgt bei 60°C Ölbadtemperatur. Man erhält 2.66 g 1-Butyl-3-methylimidazolium Benzolsulfonat in annähernd quantitativer Ausbeute.

$^1H$ NMR (Referenz: TMS; $CD_3CN$), ppm: 0.89 t ($CH_3$); 1.27 m ($CH_2$); 1.76 m ($CH_2$); 3.82 s ($CH_3$); 4.12 t ($CH_2$); 7.32-7.39 (3H, $C_6H_5$); 7.39 d,d (CH); 7.43 d,d (CH); 7.70-7.79 (2H, $C_6H_5$); 9.01 br. s. (CH); $^3J_{H,H}$ = 7.4 Hz; $^3J_{H,H}$ = 7.3 Hz; $J_{H,H}$ = 1.8 Hz.

Beispiel 14:

Synthese von 1-Butyl-3-methylimidazolium Trifluoracetat

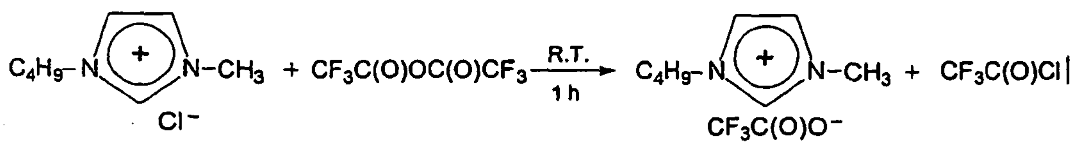


Eine Mischung von 2.42 g (13.85 mmol) 1-Butyl-3-methylimidazoliumchlorid und 3.21 g (15.28 mmol) Trifluoressigsäureanhydrid, $CF_3C(O)OC(O)CF_3$, werden bei Raumtemperatur für 3 Stunden gerührt. NMR-Messungen zeigen die Vollständigkeit der Reaktion. Der Rückstand wird für 30 Minuten im Vakuum bei 13.3 Pa und 60°C (Ölbadtemperatur) getrocknet. Man erhält 3.49 g 1-Butyl-3-methylimidazolium Trifluoracetat in annähernd quantitativer Ausbeute.

$^1H$ NMR (Referenz: TMS; $CD_3CN$), ppm: 0.89 t ($CH_3$); 1.27 m ($CH_2$); 1.78 m ($CH_2$); 3.84 s ($CH_3$); 4.15 t ($CH_2$); 7.44 d,d (CH); 7.49 d,d (CH); 9.24 br. s. (CH); $^3J_{H,H}$ = 7.4 Hz; $^3J_{H,H}$ = 7.1 Hz; $J_{H,H}$ = 1.7 Hz.
$^{19}F$ NMR (Referenz: $CCl_3F$ - intern; $CD_3CN$), ppm: -74.4 s ($CF_3$).

Beispiel 15:

Analog zu Beispiel 14 werden
1,3-Dimethylimidazoliumchlorid mit Trifluoressigsäureanhydrid zu 1,3-Dimethytimidazolium Trifluoracetat;

1-Butyl-1-methylpyrrolidiniumchlorid mit Trifluoressigsäureanhydrid zu 1-Butyl-1-methylpyrrolidinium Trifluoracetat oder Methyltrioctylammoniumchlorid mit Trifluoressigsäureanhydrid zu Methyltrioctylammonium Trifluoracetat umgesetzt.

Beispiel 16:

Synthese von 1-Ethyl-3-methylimidazolium p-Toluensulfonat (Tosylat)

a) Synthese von 1-Ethyl-3-methylimidazoliumbromid

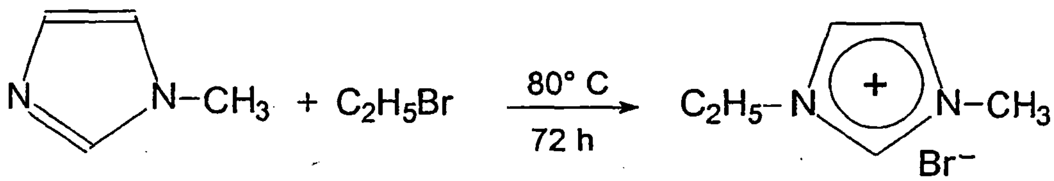


Es werden 111.43 g (1.36 mol) Methylimidazol und 160 g (1.47 mol) Bromethan vermischt und anschließend 400 ml Isopropanol zugegeben. Die Reaktionsmischung wird unter Rühren für 72 Stunden erhitzt, wobei die Ölbadtemperatur 80°C beträgt. Isopropanol wird danach abdestilliert und der Rückstand im Vakuum bei 13.3 Pa und 100°C Ölbadtemperatur für zwei Stunden getrocknet. Man erhält 258.6 g 1-Ethyl-3-methylimidazoliumbromid, das entspricht einer Ausbeute von 99.7 %.

Smp.: 73-74°C.

$^1H$ NMR (Referenz: TMS; $CD_3CN$), ppm: 1.44 t ($CH_3$); 3.88 s ($CH_3$); 4.23 q ($CH_2$); 7.49 m (CH); 7.56 m (CH); 9.45 br. s. (CH); $^3J_{H,H}$ = 7.2 Hz.

b) Synthese von 1-Ethyl-3-methylimidazolium Tosylat

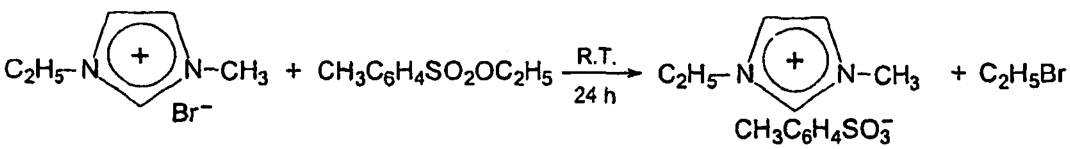


Zu 43.41 g (0.227 mol) 1-Ethyl-3-methylimidazoliumbromid werden 45.5 g (0.227 mol) geschmolzenes Ethyltosylat (p-$CH_3C_6H_4SO_2OC_2H_5$, Smp. 32-34°C) gegeben. Die Reaktionsmischung wird 24 Stunden bei Raumtemperatur gerührt, bis sich das Bromidsalz vollständig gelöst hat. Das flüssige Produkt wird anschließend im Vakuum bei 13.3 Pa und 60°C Ölbadtemperatur für eine Stunde getrocknet. Man erhält 64.1 g 1-Ethyl-3-methylimidazolium Tosylat, das entspricht einer annähernd quantitativen Ausbeute.

$^1H$ NMR (Referenz: TMS; $CD_3CN$), ppm: 1.35 t ($CH_3$); 2.29 s ($CH_3$); 3.78 s ($CH_3$); 4.11 q ($CH_2$); 7.13 d (2CH, A); 7.60 d (2CH, B); 7.42 m (CH); 7.49 m (CH); 9.10 br. s. (CH); $^3J_{H,H}$ = 7.3 Hz; $^3J_{H(A),H(B)}$ = 8.1 Hz.

Beispiel 17:

Analog zu Beispiel 16 werden
1-Methylimidazoliumchlorid mit Trimethylsilyl(p-toluen)sulfonat zu 1-Methylimidazolium p-Toluensulfonat;
1-Butyl-imidazoliumchlorid mit Trimethylsilyl(p-toluen)sulfonat zu 1-Butyl-imidazolium p-Toluensulfonat;
1-Butyl-3-methylimidazoliumchlorid mit Trimethylsilyl(p-toluen)sulfonat zu 1-Butyl-3-methylimidazolium p-Toluensulfonat;
1-Butyl-2,3-dimethylimidazoliumchlorid mit Trimethylsilyl(p-toluen)sulfonat zu
1-Butyl-2,3-dimethylimidazolium p-Toluensulfonat oder

Tri-isobutyl-methyl-phosphoniumbromid mit Trimethylsilyl(p-toluen)sulfonat zu
Tri-isobutyl-methyl-phosphonium p-Toluensulfonat umgesetzt.

Beispiel 18:

Synthese von N,N,N',N'-Tetramethyl-N''-ethylguanidinium Trifluormethansulfonat

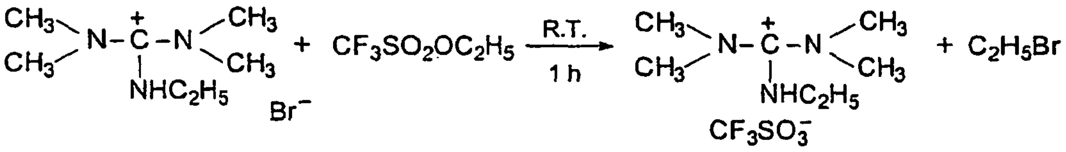


Eine Mischung von 2.38 g (10.62 mmol) N,N,N',N'-Tetramethyl-N''-ethylguanidiniumbromid und 1.95 g (10.95 mmol) Ethyltrifluormethansulfonat werden bei Raumtemperatur für eine Stunde gerührt. Eine NMR-Messung zeigt die Vollständigkeit der Reaktion. Der Rückstand wird anschließend im Vakuum von 13.3 Pa und 60°C Ölbadtemperatur für 2 Stunden getrocknet. Man erhält 3.11 g N,N,N',N'-Tetramethyl-N''-ethylguanidinium Trifluormethansulfonat als Flüssigkeit, das entspricht einer annähernd quantitativen Ausbeute.

$^1$H NMR (Referenz: TMS; $CD_3CN$), ppm: 1.13 t ($CH_3$); 2.87 br.s ; 2.89 br.s ; 2.92 s ($4CH_3$); 3.22 m ($CH_2$); 6.42 br.s (NH); $^3J_{H,H}$ = 7.1 Hz.

$^{19}$F NMR (Referenz: $CCl_3F$ - intern; $CD_3CN$), ppm: -78.0 s ($CF_3$).

Analog hierzu werden
N,N,N',N',N''-Pentamethyl-N''-propylguanidiniumchlorid mit Methyl- oder Ethyltriflat zu
N,N,N',N',N''-Pentamethyl-N''-propylguanidinium Triflat;
Hexamethylguanidiniumchlorid mit Methyl- oder Ethyltriflat zu
Hexamethylguanidinium Triflat oder
S-Ethyl-N,N,N',N'-tetramethylisothiouroniumiodid mit Methyl- oder
Ethyltriflat T zu
S-Ethyl-N,N,N',N'-tetramethylisothiouronium Triflat umgesetzt.

Beispiel 19:

Synthese von 1-Cyano-4-dimethylaminopyridinium Trifluormethansulfonat

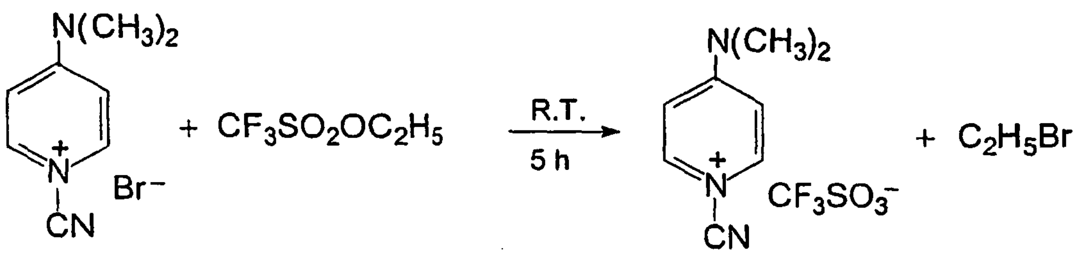


Eine Mischung von 1.88 g (8.24 mmol) 1-Cyano-4-dimethylaminopyridiniumbromid und 1.93 g (10.83 mmol) Ethyltrifluormethansulfonat werden bei Raumtemperatur für 5 Stunden gerührt. Anschließend werden die flüchtigen Verbindungen im Vakuum entfernt und der Rückstand bei Raumtemperatur im Vakuum von 13.3 Pa 2 Stunden getrocknet. Man erhält 2.40 g 1-Cyano-4-dimethylaminopyridinium Trifluormethansulfonat, das entspricht einer Ausbeute von 98%.

Smp.: 75-77°C
$^1$H NMR (Referenz: TMS; $CD_3CN$), ppm 3.33 s ($2CH_3$); 7.02 d,m (2CH, A); 8.09 d,m (2CH, B); $^3J_{H(A),H(B)}$ = 8.1 Hz.
$^{19}$F NMR (Referenz: $CCl_3F$-intern; $CD_3CN$), ppm: -78.02 s ($CF_3$).
$^{13}$C NMR (Referenz: TMS; $CD_3CN$), ppm: 42.2 q,q [$N(CH_3)_2$]; 107.6 m (CN); 109.8 d,m (2CH); 141.5 d,m (2CH); 158.0 m (C); $^1J_{C,H}$ = 195 Hz ; $^1J_{C,H}$ =

175 Hz ; $^1J_{C,H}$ = 142 Hz ; $^3J_{C,H}$ = 3.3 Hz.

Raman-Spektrum: 2272.9 $cm^{-1}$ (CN).
Elementaranalyse $C_9H_{10}F_3N_3O_3S$ (Molmasse 297.25):
gefunden: C 36.00 %, H 3.22 %, N 13.92 %, S 9.84 %;
berechnet: C 36.37 %, H 3.39 %, N 14.14 %, S 10.79 %.

Beispiel 20:

Synthese von 1-Ethyl-2,3-dimethylimidazolium p-Toluensulfonat

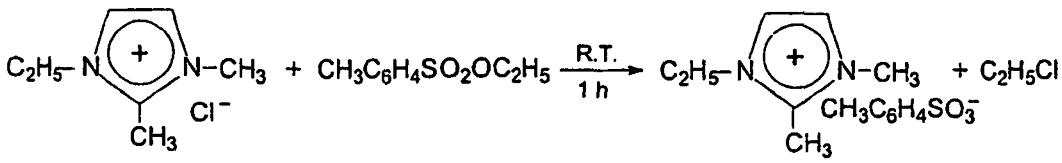


Zu 2.08 g (12.95 mmol) 1-Ethyl-2,3-dimethylimidazoliumbromid, werden 2.65 g (13.23 mmol) geschmolzenes Ethyltosylat (Schmelzpunkt 32-34°C) zugegeben. Die Reaktionsmischung wird bei Raumtemperatur für 1 Stunde gerührt und im Vakuum bei 13.3 Pa und 110-112°C Ölbadtemperatur getrocknet. Man erhält 3.83 g 1-Ethyl-2,3-dimethylimidazolium p-Toluensulfonat. Die Ausbeute ist annähernd quantitativ.

$^1$H NMR (Referenz: TMS; $CD_3CN$), ppm: 1.33 t ($CH_3$); 2.32 s ($CH_3$); 2.48 s ($CH_3$); 3.68 s ($CH_3$); 4.07 q ($CH_2$); 7.13 d (2CH, A); 7.47 d (2CH, B); 7.36 m (2CH); $^3J_{H,H}$ = 7.3 Hz; $^3J_{H(A),H(B)}$ = 8.0 Hz.

Beispiel 21:

Synthese von 1-Ethyl-1-methylpyrrolidinium Triflat

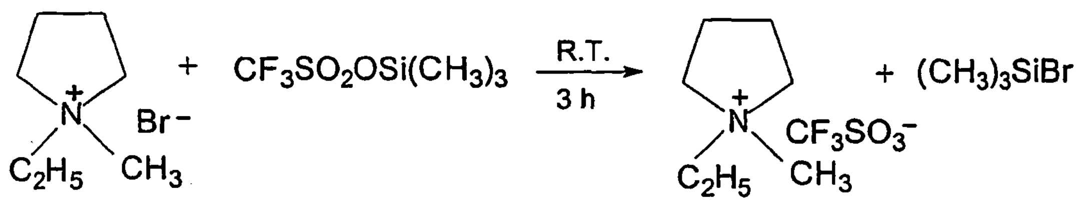


Eine Mischung von 2.36 g (12.15 mmol) 1-Ethyl-1-methylpyrrolidiniumbromid und 2.70 g (12.15 mmol) Trimethylsilyltriflat werden für 3 Stunden bei Raumtemperatur gerührt. Alle flüchtigen Produkte werden im Vakuum bei 13.3 Pa und 60°C innerhalb einer Stunde entfernt. Man erhält 3.19 g 1-Ethyl-1-methylpyrrolidinium Triflat. Die Ausbeute ist annähernd quantitativ.

Smp. 94-95°C
$^1$H NMR (Referenz: TMS; $CD_3CN$), ppm: 1.32 t,m ($CH_3$); 2.15 m ($2CH_2$); 2.95 s ($CH_3$); 3.35 q ($CH_2$); 3.42 m ($2CH_2$); $^3J_{H,H}$ = 7.3 Hz.
$^{19}$F NMR (Referenz: $CCl_3F$-intern; $CD_3CN$), ppm: -78.03 s ($CF_3$).

Analog dazu werden
1-Butyl-1-methylpyrrolidiniumchlorid mit Trimethylsilyltriflat zu 1-Butyl-1-methylpyrrolidinium Triflat umgesetzt.

Beispiel 22:

Synthese von 1-Methyl-imidazolium Triflat

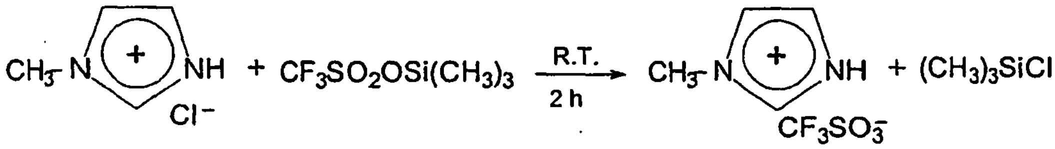


Eine Mischung von 2.23 g (18.80 mmol) 1-Methylimidazoliumchlorid und 4.61 g (20.74 mmol) Trimethylsilyltriflat werden für 2 Stunden bei Raumtemperatur gerührt. NMR-Messungen zeigen die Vollständigkeit der Reaktion. Der Rückstand wird für 1 Stunde im Vakuum bei 13.3 Pa und 60°C Ölbadtemperatur getrocknet. Man erhält 4.36 g 1-Methylimidazolium Triflat. Die Ausbeute ist annähernd quantitativ.
Smp. 90-91°C
$^1H$ NMR (Referenz: TMS; $CD_3CN$), ppm: 3.86 s ($CH_3$); 7.38 br.s (2CH); 8.54 br.s (CH); 12.30 br. s (NH).
$^{19}F$ NMR (Referenz: $CCl_3F$ - intern; $CD_3CN$), ppm: -78.1 s ($CF_3$).

Beispiel 23:

Synthese von 1-Ethyl-3-methylimidazoliumtrifluoroacetat

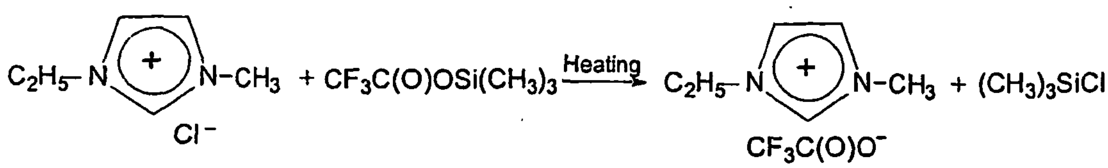


Eine Mischung aus 19.14 g (131 mmol) 1-Ethyl-3-methylimidazoliumchlorid und 72.62 g (390 mmol) Trimethylsilyltrifluoroacetat, $CF_3C(O)OSi(CH_3)_3$, werden erhitzt und für drei Stunden refluxiert. Trimethylchlorosilan, $(CH_3)_3SiCl$, das sich während der Reaktion bildet und überschüssiges Trimethylsilyltrifluoroacetat werden über eine Kolonne abdestilliert. Der Rückstand wird bei 13.3 Pa und bei einer Badtemperatur von 80°C innerhalb von 30 Minuten umgepumpt. Man erhält 29.25 g 1-Ethyl-3-methylimidazoliumtrifluoroacetat. Die Ausbeute ist nahezu quantitativ.
$^1H$ NMR (Referenz: TMS; $CD_3CN$), ppm: 1.41 t ($CH_3$); 3.82 s ($CH_3$); 4.16 q ($CH_2$); 7.38 d,d (CH); 7.44 d,d (CH); 8.79 br. s. (CH); $^3J_{H,H}$ = 7.3 Hz; $J_{H,H}$ = 1.7 Hz.
$^{19}F$ NMR (Referenz: $CCl_3F$ - intern; $CD_3CN$), ppm: -74.5 s ($CF_3$).

## Patentansprüche

**1.** Verfahren zur Herstellung von Onium-Salzen mit Alkyl- oder Arylsulfonat-Anionen oder Alkyl- oder Arylcarboxylat-Anionen durch Umsetzung eines Onium-Halogenids der Formel (1),

$[NR_4]^+ Hal^-$ (1),

oder
der Formel (2),

$[PR_4]^+ Hal^-$ (2),

wobei

Hal Cl, Br oder I und
R jeweils unabhängig voneinander
H, wobei nicht alle Substituenten R gleichzeitig H sein dürfen, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann bedeutet,
wobei ein oder mehrere R teilweise oder vollständig mit -F substituiert sein können, wobei jedoch nicht alle vier oder drei R vollständig mit F substituiert sein dürfen,
und wobei ein oder zwei nicht benachbarte und nicht α- oder ω-ständige Kohlenstoffatome des R, durch Atome und/oderAtomgruppierungen ausgewählt aus der Gruppe -O-, -S-, -S(O)- oder $-SO_2-$ ersetzt sein können
oder
der Formel (3),

$$[(R^1R^2N)\text{-}C(=SR^7)(NR^3R^4)]^+ Hal^- \quad (3),$$

wobei
Hal Cl, Br oder I und
$R^1$ bis $R^7$ jeweils unabhängig voneinander
Wasserstoff oder CN, wobei Wasserstoff für $R^7$ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten $R^1$ bis $R^7$ teilweise oder vollständig mit -F substituiert sein können, wobei jedoch nicht alle Substituenten an einem N-Atom vollständig mit F substituiert sein dürfen, wobei die Substituenten $R^1$ bis $R^7$ paarweise durch Einfach- oder Doppelbindung miteinander verbunden sein können
und wobei ein oder zwei nicht benachbarte, nicht direkt am Heteroatom gebundene und nicht ω-ständige Kohlenstoffatome der Substituenten $R^1$ bis $R^7$ durch Atome und/oder Atomgruppierungen ausgewählt aus der Gruppe -O-, -S-, -S(O)- oder$-SO_2-$ ersetzt sein können
oder
dass das Halogenid der Formel (4) entspricht,

$$[C(NR^1R^2)(NR^3R^4)(NR^5R^6)]^+ Hal^- \quad (4),$$

wobei,
Hal Cl, Br oder I und
$R^1$ bis $R^6$ jeweils unabhängig voneinander
Wasserstoff oder CN,
geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten $R^1$ bis $R^6$ teilweise oder vollständig mit -F substituiert sein können, wobei jedoch nicht alle Substituenten an einem N-Atom vollständig mit F substituiert sein dürfen, wobei die Substituenten $R^1$ bis $R^6$ paarweise durch Einfach- oder Doppelbindung miteinander verbunden sein können
und wobei ein oder zwei nicht benachbarte, nicht direkt am Heteroatom gebundene und nicht ω-ständige Kohlenstoffatome der Substituenten $R^1$ bis $R^6$ durch Atome und/oder Atomgruppierungen ausgewählt aus der Gruppe -O-, -S-, -S(O)- oder$-SO_2-$ ersetzt sein können
oder
der Formel (5),

$$[HetN]^+ Hal^- \quad (5)$$

wobei

Hal Cl, Br oder I und
HetN$^+$ ein heterocyclisches Kation, ausgewählt aus der Gruppe

Imidazolium
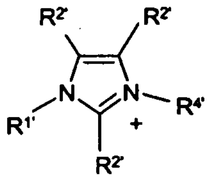
,
1H-Pyrazolium
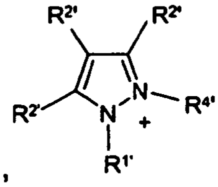
,
3H-Pyrazolium
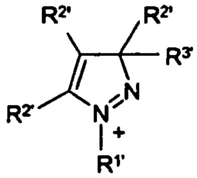
,
4H-Pyrazolium
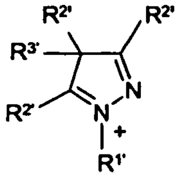
,
1-Pyrazolinium
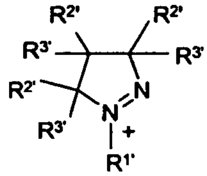
,
2-Pyrazolinium
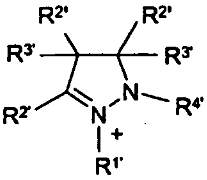
,
3-Pyrazolinium
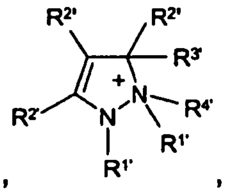
,
2,3-Dihydro-Imidazolinium
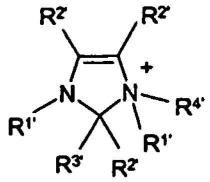
,
4,5-Dihydro-Imidazolinium
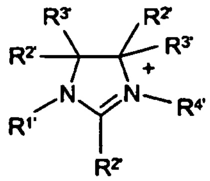
,
2,5-Dihydro-Imidazolinium
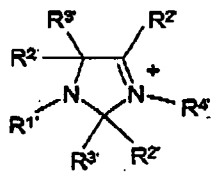
,
Pyrrolidinium
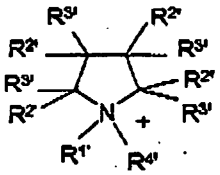
,
1,2,4-Triazolium
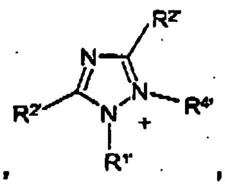
,
1,2,4-Triazolium
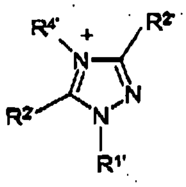
,
1,2,3-Triazolium
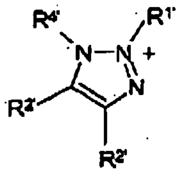
,
1,2,3-Triazolium
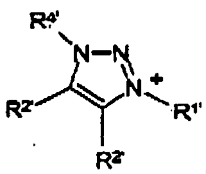
,
Pyridinium
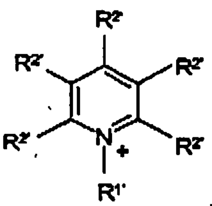
,
Pyridazinium
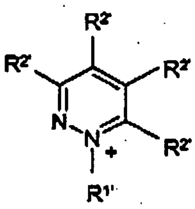
,
Pyrimidinium
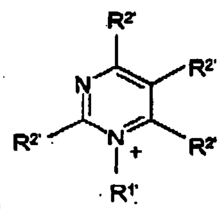
,
Piperidinium
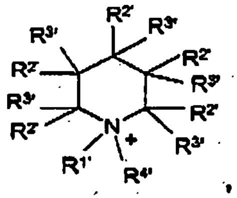
,
Morpholinium
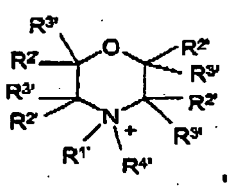
,
Pyrazinium
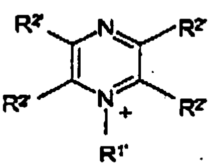
,
Thiazolium
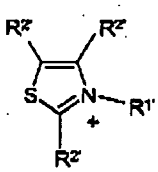
oder
Oxazolium
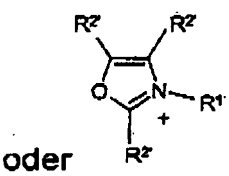

bedeutet, wobei die Substituenten
$R^{1'}$ bis $R^{4'}$ jeweils unabhängig voneinander
Wasserstoff oder CN,

geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7 C-Atomen, das mit Alkylgruppen mit 1-6 C-Atomen substituiert sein kann oder Aryl-$C_1$-$C_6$-alkyl bedeutet,
wobei die Substituenten $R^{1'}$ und $R^{4'}$ teilweise oder vollständig mit F substituiert sein können, wobei jedoch nicht gleichzeitig $R^{1'}$ und $R^{4'}$ CN sind oder vollständig mit F substituiert sein dürfen,
wobei die Substituenten $R^{2'}$ und $R^{3'}$ teilweise oder vollständig mit halogenen oder teilweise mit $NO_2$ oder CN substituiert sein dürfen und wobei ein oder zwei nicht benachbarte, nicht direkt am Heteroatom gebundene und nicht ω-ständige Kohlenstoffatome der Substituenten $R^{1'}$ bis $R^{4'}$, durch Atome und/oder Atomgruppierungen ausgewählt aus der Gruppe -O-, -S-, -S(O)- oder -$SO_2$- ersetzt sein können,
mit einem Alkyl- oder Trialkylsilylester einer Alkyl- oder Arylsulfonsäure oder einer Alkyl- oder Arylcarbonsäure, ausgewählt aus Methyltrifluormethansulfonat, Ethyltrifluormethansulfonat, Propyltrifluormethansulfonat, Butyltrifluormethansulfonat, Methylmethansulfonat, Ethylmethansulfonat, Propylmethansulfonat, Butylmethansulfonat, Methylethansulfonat, Ethylethansulfonat, Propylethansulfonat, Butylethansulfonat, Methylbenzolsulfonat, Ethylbenzolsulfonat, Methyl-p-toluensulfonat, Ethyl-p-toluensulfonat, Propyl-p-toluensulfonat, Methyl-p-nitrobenzolsulfonat oder Ethyl-p-nitroenzolsulfonat,
Methylchlorsulfonat oder Trimethylsilylchlorsulfonat,
$CF_3$-$SO_2$-O-$Si(CH_3)_3$, $CF_3$-$SO_2$-O-$Si(CH_3)_2(t$-$C_4H_9)$, $CF_3$-$SO_2$-O-$Si(C_2H_5)_2(i$-$C_3H_7)$, $CF_3$-$SO_2$-O-$Si(C_2H_5)_3$, $CF_3$-$SO_2$-O-$Si(i$-$C_3H_7)_3$, $CF_3$-$SO_2$-O-Si-$(n$-$C_4H_9)_3$, $CH_3SO_2$-O-$Si(CH_3)_3$, $CH_3$-$SO_2$-O-$Si(CH_3)_2(n$-$C_4H_9)$, $(C_2H_5)$-$SO_2$-O-Si-$(CH_3)_3$, $(C_2H_5)$-$SO_2$-O-$Si(C_2H_5)_3$, $C_2F_5$-$SO_2$-O-$Si(CH_3)_3$, $C_4F_9SO_2$-O-$Si(CH_3)_3$, $C_4F_9$-$SO_2$-O-Si $(C_2H_5)_3$, $C_4F_9$-$SO_2$-O-$Si(CH_3)_2(n$-$C_4H_9)$, $C_4F_9$-$SO_2$-O-$Si(CH_3)(n$-$C_4H_9)_2$, $C_8F_{17}$-$SO_2$-O-$Si(CH_3)_3$, $C_6F_5$-$SO_2$-O-Si $(CH_3)_3$ oder $(p$-$CH_3)C_6H_4$-$SO_2$-O-$Si(CH_3)_3$, Methyltrifluoracetat, Ethyltrifluoracetat, Propyltrifluoracetat, Butyltrifluoracetat, tert-Butylacetat, tert-Butylpropionat, iso-Propylbenzoat, tert-Butylbenzoat, Methyl-4-nitrobenzoat oder Methylpentafluorbenzoat,
$CF_3$-C(O)-O-$Si(CH_3)_3$, $CF_3$-C(O)-O-$Si(CH_3)_2(t$-$C_4H_9)$, $CF_3$-C(O)-O-$Si(C_2H_5)_2(i$-$C_3H_7)$, $CF_3$-C(O)-O-$Si(C_2H_5)_3$, $CF_3$-C(O)-O-$Si(i$-$C_3H_7)_3$, $CF_3$-C(O)-O-Si-$(n$-$C_4H_9)_3$, $CH_3$-C(O)-O-$Si(CH_3)_3$, $CH_3$-C(O)-O-$Si(CH_3)_2(n$-$C_4H_9)$, $(C_2H_5)$-C(O)-O-Si-$(CH_3)_3$, $(C_2H_5)$-C(O)-O-$Si(C_2H_5)_3$, $C_2F_5$-C(O)-O-$Si(CH_3)_3$, $C_4F_9$-C(O)-O-$Si(CH_3)_3$, $C_4F_9$-C (O)-O-$Si(C_2H_5)_3$, $C_4F_9$-C(O)-O-$Si(CH_3)_2(n$-$C_4H_9)$, $C_4F_9$-C(O)-O-$Si(CH_3)(n$-$C_4H_9)_2$, $C_8F_{17}$-C(O)-O-$Si(CH_3)_3$, $C_6H_5$-C(O)-O-$Si(CH_3)_3$ oder $(p$-$NO_2)C_6H_4$-C(O)-O-$Si(CH_3)_3$ oder $(C_6F_5$-C(O)-O-$Si(CH_3)_3)$,
bei Temperaturen zwischen 10° und 30°C, ohne Einsatz eines Lösungsmittels,

**2.** Verwendung des Verfahrens nach Anspruch 1 zur Aufreinigung von durch Onlum-Halogenide verunreinigten ionischen Flüssigkeiten mit Alkyl- oder Arylsulfonat-Anionen oder Alkyl- oder Arylcarboxylat-Anionen.

**3.** Trifluormethansulfonate der Formel (11)

$$HetN^+ [CF_3SO_3]^- \qquad (11),$$

wobei
$HetN^+$ ein heterocyclisches Kation, ausgewählt aus der Gruppe

Imidazolium 1H-Pyrazolium 3H-Pyrazolium 4H-Pyrazolium 1-Pyrazolinium

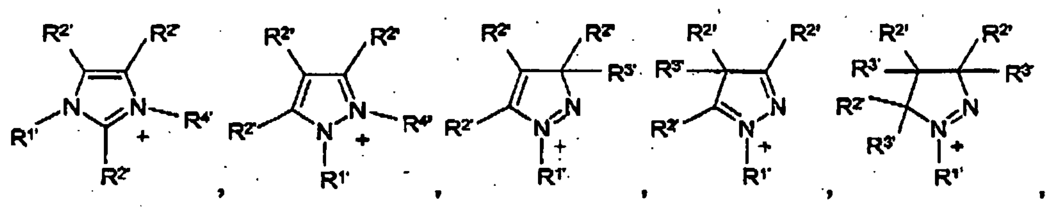

, , , , ,

2-Pyrazolinium
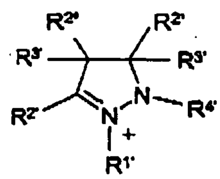
,
3-Pyrazolinium
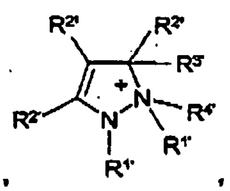
,
2,3-Dihydro-Imidazolinium
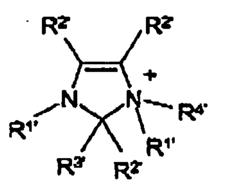
,
4,5-Dihydro-Imidazolinium
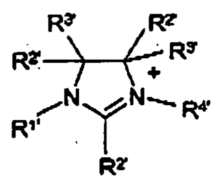
,
2,5-Dihydro-Imidazolinium
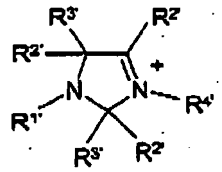
,
Pyrrolidinium
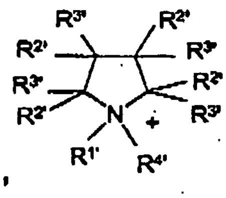
,
1,2,4-Triazolium
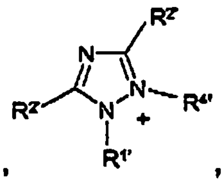
,
1,2,4-Triazolium
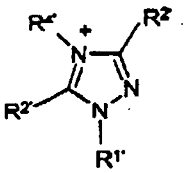
,
1,2,3-Triazolium
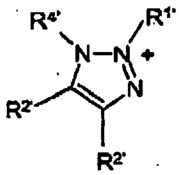
,
1,2,3-Triazolium
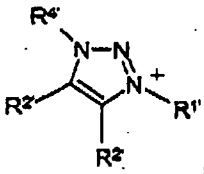
,
Pyridinium
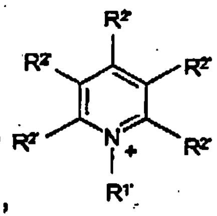
,
Pyridazinium
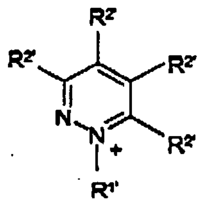
,
Pyrimidinium
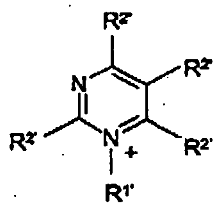
,
Piperidinium
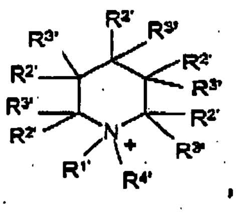
,
Morpholinium
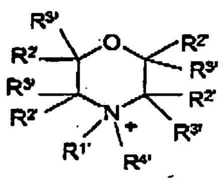
,
Pyrazinium
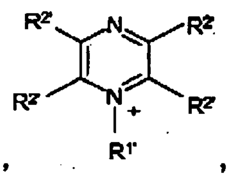
,
Thiazolium
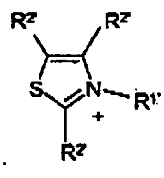
oder
Oxazolium
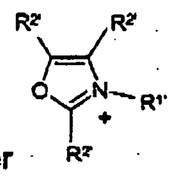

bedeutet, wobei
$R^{1'}$ oder $R^{4'}$ CN bedeutet und die übrigen Substituenten
$R^{1'}$ bis $R^{4'}$ jeweils unabhängig voneinander
Wasserstoff,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7 C-Atomen, das mit Alkylgruppen mit 1-6 C-Atomen substituiert sein kann oder Aryl-$C_1$-$C_6$-alkyl bedeutet,
wobei ein oder mehrere Substituenten $R^{2'}$ und $R^{3'}$ teilweise oder vollständig mit -F substituiert sein können,
wobei jedoch nicht gleichzeitig $R^{1'}$ und $R^{4'}$ CN sind
und wobei ein oder zwei nicht benachbarte, nicht direkt am Heteroatom gebundene und nicht ω-ständige Kohlenstoffatome der Substituenten $R^{1'}$ bis $R^{4'}$, durch Atome und/oder Atomgruppierungen ausgewählt aus der Gruppe -O-, -S-, -S(O)- oder -$SO_2$- ersetzt sein können.

**4.** Verwendung der Verbindungen der Formel (11) als Cyanierungsreagenz.

## Claims

**1.** Process for the preparation of onium salts with alkyl- or arylsulfonate anions or alkyl- or arylcarboxylate anions by reaction of an onium halide of the formula (1)

$$[NR_4]^+ \; Hal^- \qquad (1),$$

or
of the formula (2)

$$[PR_4]^+ \; Hal^- \qquad (2),$$

where
Hal denotes Cl, Br or I and
R in each case, independently of one another, denotes
H, where all substituents R cannot simultaneously be H,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more R may be partially or fully substituted by -F, but
where all four or three R cannot be fully substituted by F,
and where, in the R, one or two non-adjacent carbon atoms which are not in the α- or ω-position may be replaced by atoms and/or atom groups selected from the group -O-, -S-, -S(O)- or $-SO_2-$,
or
of the formula (3)

$$[(R^1R^2N)\text{-}C(=SR^7)(NR^3R^4)]^+ \; Hal^- \qquad (3),$$

where
Hal denotes Cl, Br or I and
$R^1$ to $R^7$ each, independently of one another, denote
hydrogen or CN, where hydrogen is excluded for $R^7$,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more of the substituents $R^1$ to $R^7$ may be partially or fully substituted by -F, but where all substituents on an N atom cannot be fully substituted by F,
where the substituents $R^1$ to $R^7$ may be connected to one another in pairs by a single or double bond
and where, in the substituents $R^1$ to $R^7$, one or two non-adjacent carbon atoms which are not bonded directly to the heteroatom and are not in the
ω-position may be replaced by atoms and/or atom groups selected from the group -O-, -S-, -S(O)- or $-SO_2-$,
or
of the formula (4)

$$[C(NR^1R^2)(NR^3R^4)(NR^5R^6)]^+ \; Hal^- \qquad (4),$$

where
Hal denotes Cl, Br or I and
$R^1$ to $R^6$ each, independently of one another, denote
hydrogen or CN,
straight-chain or branched alkyl having 1 to 20 C atoms,

straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more of the substituents $R^1$ to $R^6$ may be partially or fully substituted by -F, but where all substituents on an N atom cannot be fully substituted by F,
where the substituents $R^1$ to $R^6$ may be connected to one another in pairs by a single or double bond
and where, in the substituents $R^1$ to $R^6$, one or two non-adjacent carbon atoms which are not bonded directly to the heteroatom and are not in the
ω-position may be replaced by atoms and/or atom groups selected from the group -O-, -S-, -S(O)- or $-SO_2-$,
or
of the formula (5)

$$[HetN]^+ \ Hal^- \qquad (5),$$

where
Hal denotes Cl, Br or I and
$HetN^+$ denotes a heterocyclic cation selected from the group

imidazolium

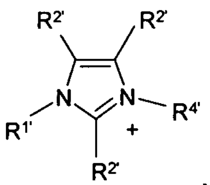

,

1H-pyrazolium

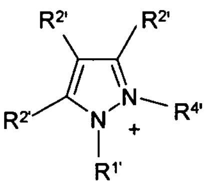

,

3H-pyrazolium

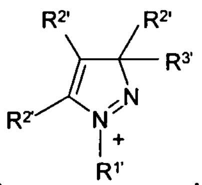

,

4H-pyrazolium

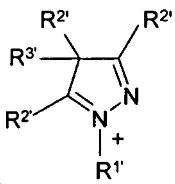

,

1-pyrazolinium

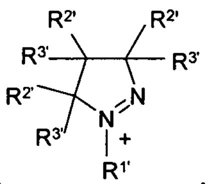

,

2-pyrazolinium

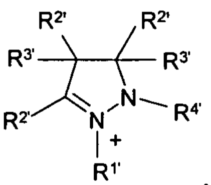

,

3-pyrazolinium

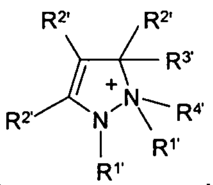

,

2,3-dihydroimidazolinium

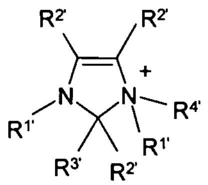

,

4,5-dihydroimidazolinium

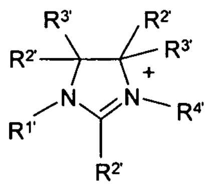

,

2,5-dihydroimidazolinium

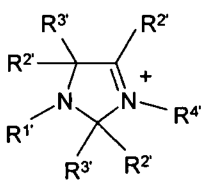

,

pyrrolidinium

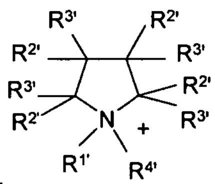

,

1,2,4-triazolium

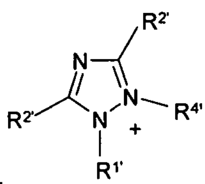

,

1,2,4-triazolium

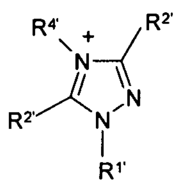

,

1,2,3-triazolium 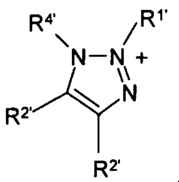 , 1,2,3-triazolium 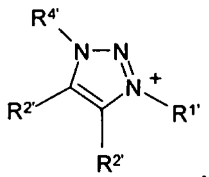 , pyridinium 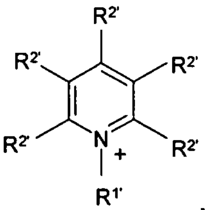 , pyridazinium 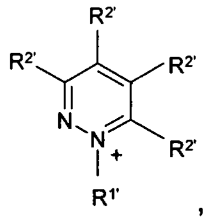 , pyrimidinium 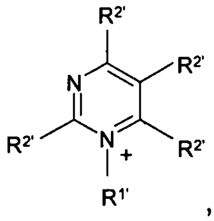 ,

piperidinium 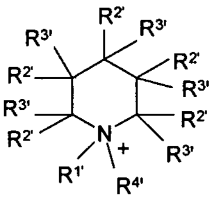 , morpholinium 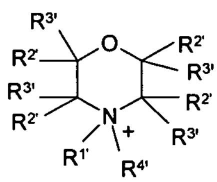 , pyrazinium 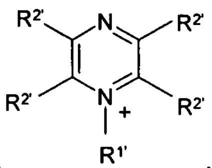 , thiazolium 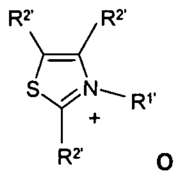 or oxazolium 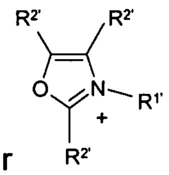

where the substituents
$R^{1'}$ to $R^{4'}$ each, independently of one another, denote
hydrogen or CN,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
dialkylamino having alkyl groups having 1-4 C atoms, but which is not bonded to the heteroatom of the heterocycle,
saturated, partially or fully unsaturated cycloalkyl having 3-7 C atoms, which may be substituted by alkyl groups having 1-6 C atoms,
or aryl-$C_1$-$C_6$-alkyl,
where the substituents $R^{1'}$ and $R^{4'}$ may be partially or fully substituted by F, but where $R^{1'}$ and $R^{4'}$ are not simultaneously CN or cannot simultaneously be fully substituted by F,
where the substituents $R^{2'}$ and $R^{3'}$ may be partially or fully substituted by halogens or partially substituted by $NO_2$ or CN and where, in the substituents $R^{1'}$ to $R^{4'}$, one or two non-adjacent carbon atoms which are not bonded directly to the heteroatom and are not in the ω-position may be replaced by atoms and/or atom groups selected from the group -O-, -S-, -S(O)- or $-SO_2-$,
with an alkyl or trialkylsilyl ester of an alkyl- or arylsulfonic acid or alkyl- or arylcarboxylic acid selected from
methyl trifluoromethanesulfonate, ethyl trifluoromethanesulfonate, propyl trifluoromethanesulfonate, butyl trifluoromethanesulfonate, methyl methanesulfonate, ethyl methanesulfonate, propyl methanesulfonate, butyl methanesulfonate, methyl ethanesulfonate, ethyl ethanesulfonate, propyl ethanesulfonate, butyl ethanesulfonate, methyl benzenesulfonate, ethyl benzenesulfonate, methyl p-toluenesulfonate, ethyl p-toluenesulfonate, propyl p-toluenesulfonate, methyl p-nitrobenzenesulfonate or ethyl p-nitrobenzenesulfonate, methyl chlorosulfonate or trimethylsilyl chlorosulfonate, $CF_3$-$SO_2$-O-$Si(CH_3)_3$, $CF_3$-$SO_2$-O-$Si(CH_3)_2(t$-$C_4H_9)$, CF3-SO2-O-$Si(C_2H_5)_2(i$-$C_3H_7)$, $CF_3$-$SO_2$-O-$Si(C_2H_5)_3$, $CF_3$-$SO_2$-O-Si(i-$C_3H_7)_3$, $CF_3$-$SO_2$-O-Si-(n-$C_4H_9)_3$, $CH_3SO_2$-O-$Si(CH_3)_3$, $CH_3$-$SO_2$-O-$Si(CH_3)_2$(n-$C_4H_9$), $(C_2H_5)$-$SO_2$-O-Si-$(CH_3)_3$, $(C_2H_5)$-$SO_2$-O-$Si(C_2H_5)_3$, $C_2F_5$-$SO_2$-O-$Si(CH_3)_3$, $C_4F_9SO_2$-O-$Si(CH_3)_3$, $C_4F_9$-$SO_2$-0-Si $(C_2H_5)_3$, $C_4F_9$-$SO_2$-O-$Si(CH_3)_2$(n-$C_4H_9$), $C_4F_9$-$SO_2$-O-$Si(CH_3)$(n-$C_4H_9)_2$, $C_8F_{17}SO_2$-O-$Si(CH_3)_3$, $C_6F_5$-$SO_2$-O-Si $(CH_3)_3$ or (p-$CH_3$)$C_6H_4$-$SO_2$-O-$Si(CH_3)_3$,
methyl trifluoroacetate, ethyl trifluoroacetate, propyl trifluoroacetate, butyl trifluoroacetate, tert-butyl acetate, tert-butyl propionate, isopropyl benzoate, tert-butyl benzoate, methyl 4-nitrobenzoate or methyl pentafluorobenzoate, $CF_3$-C(O)-O-$Si(CH_3)_3$, $CF_3$-C(O)-O-$Si(CH_3)_2$(t-$C_4H_9$), $CF_3$-C(O)-O-$Si(C_2H_5)_2$(i-$C_3H_7$), $CF_3$-C(O)-O-$Si(C_2H_5)_3$, $CF_3$-C(O)-O-Si(i-$C_3H_7)_3$, $CF_3$-C(O)-O-Si-(n-$C_4H_9)_3$, $CH_3$-C(O)-O-$Si(CH_3)_3$, $CH_3$-C(O)-O-$Si(CH_3)_2$(n-$C_4H_9$), $(C_2H_5)$-C(O)-O-Si-$(CH_3)_3$, $(C_2H_5)$-C(O)-O-$Si(C_2H_5)_3$, $C_2F_5$-C(O)-O-$Si(CH_3)_3$, $C_4F_9$-C(O)-O-$Si(CH_3)_3$, $C_4F_9$-C (O)-O-$Si(C_2H_5)_3$, $C_4F_9$-C(O)-O-$Si(CH_3)_2$(n-$C_4H_9$), $C_4F_9$-C(O)-O-$Si(CH_3)$(n-$C_4H_9)_2$, $C_8F_{17}$-C(O)-O-$Si(CH_3)_3$, $C_6H_5$-C(O)-O-$Si(CH_3)_3$, (p-$NO_2$)$C_6H_4$-C(O)-O-$Si(CH_3)_3$ or ($C_6F_5$-C(O)-O-$Si(CH_3)_3$)
at temperatures between 10° and 30°C, without the use of a solvent.

**2.** Use of the process according to Claim 1 for the purification of ionic liquids with alkyl- or arylsulfonate anions or alkyl-

or arylcarboxylate anions which are contaminated by onium halides.

**3.** Trifluoromethanesulfonates of the formula (11)

$$HetN^{+}\ [CF_3SO_3]^{-} \qquad (11),$$

where
$HetN^+$ denotes a heterocyclic cation selected from the group

imidazolium

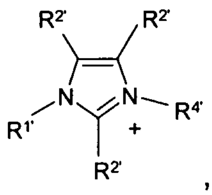

,

1H-pyrazolium

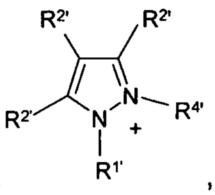

,

3H-pyrazolium

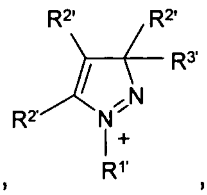

,

4H-pyrazolium

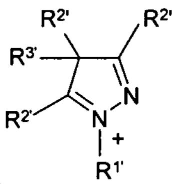

,

1-pyrazolinium

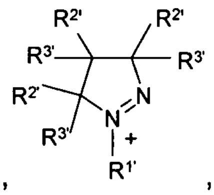

,

2-pyrazolinium

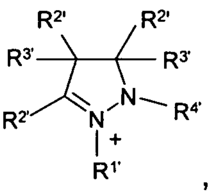

,

3-pyrazolinium

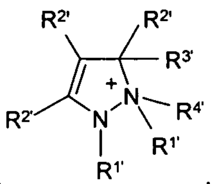

,

2,3-dihydroimidazolinium

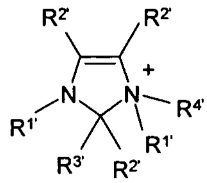

,

4,5-dihydroimidazolinium

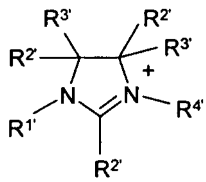

,

2,5-dihydroimidazolinium

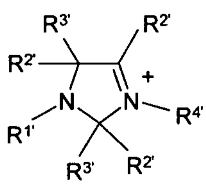

,

pyrrolidinium

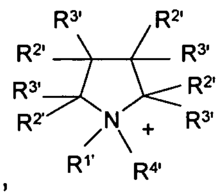

,

1,2,4-triazolium

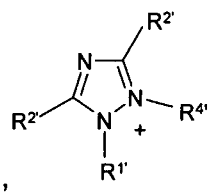

,

1,2,4-triazolium

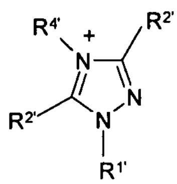

,

1,2,3-triazolium

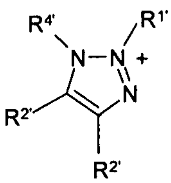

,

1,2,3-triazolium

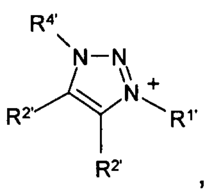

,

pyridinium

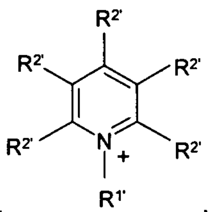

,

pyridazinium

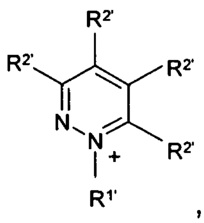

,

pyrimidinium

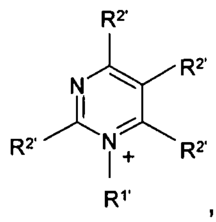

,

piperidinium

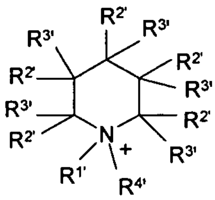

, morpholinium

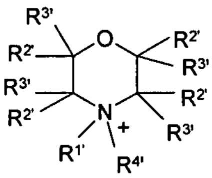

, pyrazinium

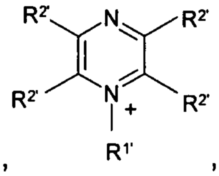

, thiazolium

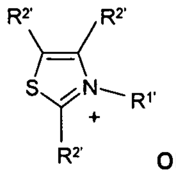

or oxazolium

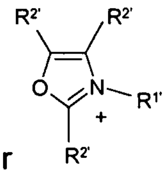

where
$R^{1'}$ or $R^{4'}$ denotes CN and the other substituents
$R^{1'}$ to $R^{4'}$ each, independently of one another, denote hydrogen,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
dialkylamino having alkyl groups having 1-4 C atoms, but which is not bonded to the heteroatom of the heterocycle,
saturated, partially or fully unsaturated cycloalkyl having 3-7 C atoms,
which may be substituted by alkyl groups having 1-6 C atoms,
or aryl-$C_1$-$C_6$-alkyl,
where one or more substituents $R^{2'}$ and $R^{3'}$ may be partially or fully substituted by -F,
but where $R^{1'}$ and $R^{4'}$ are not simultaneously CN,
and where, in the substituents $R^{1'}$ to $R^{4'}$, one or two non-adjacent carbon atoms which are not bonded directly to the heteroatom and are not in the ω-position may be replaced by atoms and/or atom groups selected from the group -O-, -S-, -S(O)- or $-SO_2-$.

4. Use of the compounds of the formula (11) as cyanating reagent.

## Revendications

1. Procédé de préparation de sels d'onium ayant des anions alkyl- ou arylsulfonate ou des anions alkyl- ou arylcarboxylate par la réaction d'un halogénure d'onium de formule (1)

$$[NR_4]^+ Hal^- \qquad (1),$$

ou
de formule (2)

$$[PR_4]^+ Hal^- \qquad (2),$$

où
Hal désigne Cl, Br ou I est
R dans chaque cas, indépendamment l'un de l'autre, désigne
H, où tous les substituants R ne peuvent être simultanément H,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
où un ou plusieurs R peuvent être partiellement ou totalement substitués par -F, mais où tous les quatre ou trois R ne peuvent être totalement substitués par F,
et où, dans R, un ou deux atomes de carbone non adjacents qui ne sont pas en position α ou ω peuvent être remplacés par des atomes et/ou des groupes d'atomes choisis parmi le groupe -O-, -S-, -S(O)- ou $-SO_2-$,
ou
de formule (3)

$[(R^1R^2N)-C(=SR^7)(NR^3R^4)]^+\ Hal^-$ (3),

où

Hal désigne Cl, Br ou I et

$R^1$ à $R^7$ chacun, indépendamment les uns des autres, désignent hydrogène ou CN, où hydrogène est exclu pour $R^7$,

alkyle à chaîne linéaire ou ramifiée ayant 1-20 atomes de C,

alcényle à chaîne linéaire ou ramifiée ayant 2-20 atomes de C et une ou plusieurs doubles liaisons,

alcynyle à chaîne linéaire ou ramifiée ayant 2-20 atomes de C et une ou plusieurs triples liaisons,

cycloalkyle saturé, partiellement ou totalement insaturé ayant 3-7 atomes de C, pouvant être substitué par des groupements alkyle ayant 1-6 atomes de C,

où un ou plusieurs parmi les substituants $R^1$ à $R^7$ peuvent être partiellement ou totalement substitués par -F, mais où tous les substituants sur un atome de N ne peuvent être totalement substitués par F,

où les substituants $R^1$ à $R^7$ peuvent être reliés les uns aux autres par paires par une liaison simple ou double

et où, dans les substituants $R^1$ à $R^7$, un ou deux atomes de carbone non adjacents qui ne sont pas liés directement à l'hétéroatome et qui ne sont

pas en position ω peuvent être remplacés par des atomes et/ou des groupes d'atomes choisis parmi le groupe -O-, -S-, -S(O)- ou $-SO_2-$,

ou

de formule (4)

$[C(NR^1R^2)(NR^3R^4)(NR^5R^6)]^+\ Hal^-$ (4),

où

Hal désigne Cl, Br ou I et

$R^1$ à $R^6$ chacun, indépendamment les uns des autres, désignent hydrogène ou CN,

alkyle à chaîne linéaire ou ramifiée ayant 1-20 atomes de C,

alcényle à chaîne linéaire ou ramifiée ayant 2-20 atomes de C et une ou plusieurs doubles liaisons,

alcynyle à chaîne linéaire ou ramifiée ayant 2-20 atomes de C et une ou plusieurs triples liaisons,

cycloalkyle saturé, partiellement ou totalement insaturé ayant 3-7 atomes de C, pouvant être substitué par des groupements alkyle ayant 1-6 atomes de C,

où un ou plusieurs parmi les substituants $R^1$ à $R^6$ peuvent être partiellement ou totalement substitués par -F, mais où tous les substituants sur un atome de N ne peuvent être totalement substitués par F,

où les substituants $R^1$ à $R^6$ peuvent être reliés les uns aux autres par paires par une liaison simple ou double

et où, dans les substituants $R^1$ à $R^6$, un ou deux atomes de carbone non adjacents qui ne sont pas liés directement à l'hétéroatome et qui ne sont

pas en position ω peuvent être remplacés par des atomes et/ou des groupes d'atomes choisis parmi le groupe -O-, -S-, -S(O)- ou $-SO_2-$,

ou

de formule (5)

$[HétN]^+\ Hal^-$ (5),

où

Hal désigne Cl, Br ou I et

$HétN^+$ désigne un cation hétérocyclique choisi parmi le groupe

imidazolium

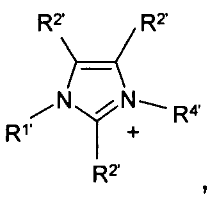

,

1H-pyrazolium

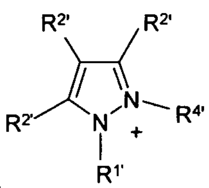

,

3H-pyrazolium

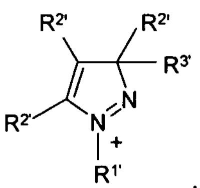

,

4H-pyrazolium

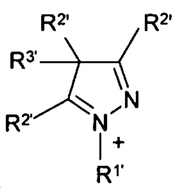

,

1-pyrazolinium

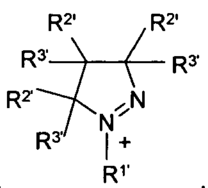

,

2-pyrazolinium

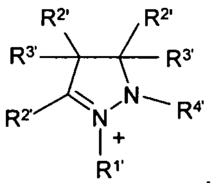

,

3-pyrazolinium

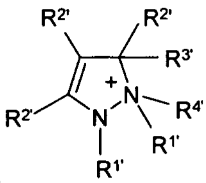

,

2,3-dihydroimidazolinium

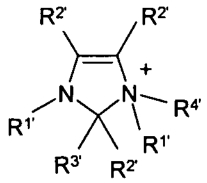

,

4,5-dihydroimidazolinium

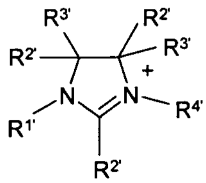

,

2,5-dihydroimidazolinium

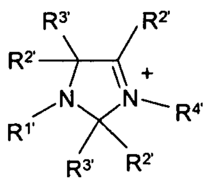

,

pyrrolidinium

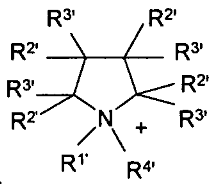

,

1,2,4-triazolium

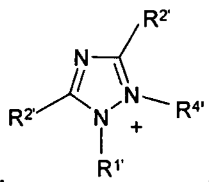

,

1,2,4-triazolium

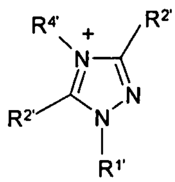

,

1,2,3-triazolium

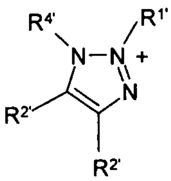

,

1,2,3-triazolium

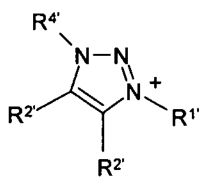

,

pyridinium

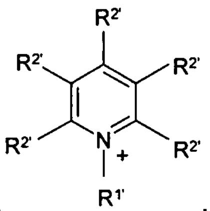

,

pyridazinium

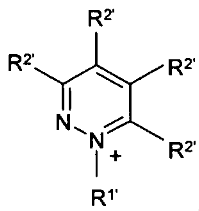

,

pyrimidinium

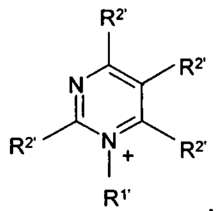

,

pipéridinium

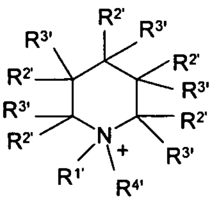

,

morpholinium

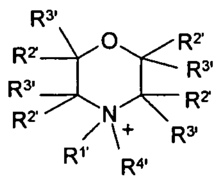

,

pyrazinium

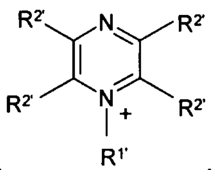

,

thiazolium

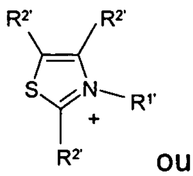

ou

oxazolium

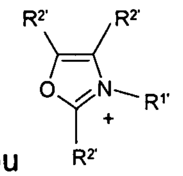

où les substituants

$R^{1'}$ à $R^{4'}$ chacun, indépendamment les uns des autres, désignent hydrogène ou CN,

alkyle à chaîne linéaire ou ramifiée ayant 1-20 atomes de C,

alcényle à chaîne linéaire ou ramifiée ayant 2-20 atomes de C et une ou plusieurs doubles liaisons,

alcynyle à chaîne linéaire ou ramifiée ayant 2-20 atomes de C et une ou plusieurs triples liaisons,

dialkylamino ayant des groupements alkyle ayant 1-4 atomes de C, mais qui n'est pas lié à l'hétéroatome de l'hétérocycle,

cycloalkyle saturé, partiellement ou totalement insaturé ayant 3-7 atomes de C, pouvant être substitué par des groupements alkyle ayant 1-6 atomes de C,

ou aryl-$C_1$-$C_6$-alkyle,

où les substituants $R^{1'}$ et $R^{4'}$ peuvent être partiellement ou totalement substitués par F, mais où $R^{1'}$ et $R^{4'}$ ne sont pas simultanément CN ou ne peuvent être simultanément totalement substitués par F,

où les substituants $R^{2'}$ et $R^{3'}$ peuvent être partiellement ou totalement substitués par des halogènes ou partiellement substitués par $NO_2$ ou CN et où, dans les substituants $R^{1'}$ à $R^{4'}$, un ou deux atomes de carbone non adjacents qui ne sont pas liés directement à l'hétéroatome et qui ne sont pas en position ω peuvent être remplacés par des atomes

et/ou des groupes d'atomes choisis parmi le groupe -O-, -S-, -S(O)- ou -$SO_2$-,
avec un alkyl- ou trialkylsilyl-ester d'un acide alkyl- ou arylsulfonique ou d'un acide alkyl- ou arylcarboxylique choisi parmi
le trifluorométhanesulfonate de méthyle, le trifluorométhanesulfonate d'éthyle, le trifluorométhanesulfonate de propyle, le trifluorométhanesulfonate de butyle, le méthanesulfonate de méthyle, le méthanesulfonate d'éthyle, le méthanesulfonate de propyle, le méthanesulfonate de butyle, l'éthanesulfonate de méthyle, l'éthanesulfonate d'éthyle, l'éthanesulfonate de propyle, l'éthanesulfonate de butyle, le benzènesulfonate de méthyle, le benzènesulfonate d'éthyle, le p-toluènesulfonate de méthyle, le p-toluènesulfonate d'éthyle, le p-toluènesulfonate de propyle, le p-nitrobenzènesulfonate de méthyle ou le p-nitrobenzènesulfonate d'éthyle, le chlorosulfonate de méthyle ou le chlorosulfonate de triméthylsilyle,
$CF_3$-$SO_2$-O-$Si(CH_3)_3$, $CF_3$-$SO_2$-O-$Si(CH_3)_2$(t-$C_4H_9$), $CF_3$-$SO_2$-O-$Si(C_2H_5)_2$(i-$C_3H_7$), $CF_3$-$SO_2$-O-$Si(C_2H_5)_3$, $CF_3$-$SO_2$-O-Si(i-$C_3H_7)_3$, $CF_3$-$SO_2$-O-Si-(n-$C_4H_9)_3$, $CH_3SO_2$-O-$Si(CH_3)_3$, $CH_3$-$SO_2$-O-$Si(CH_3)_2$(n-$C_4H_9$), $(C_2H_5)$-$SO_2$-O-Si-$(CH_3)_3$, $(C_2H_5)$-$SO_2$-O-$Si(C_2H_5)_3$, $C_2F_5$-$SO_2$-O-$Si(CH_3)_3$, $C_4F_9SO_2$-O-$Si(CH_3)_3$, $C_4F_9$-$SO_2$-O-Si $(C_2H_5)_3$, $C_4F_9$-$SO_2$-O-$Si(CH_3)_2$(n-$C_4H_9$), $C_4F_9$-$SO_2$-O-$Si(CH_3)$(n-$C_4H_9)_2$, $C_8F_{17}$-$SO_2$-O-$Si(CH_3)_3$, $C_6F_5$-$SO_2$-O-Si $(CH_3)_3$ ou (p-$CH_3$)$C_6H_4$-$SO_2$-O-$Si(CH_3)_3$, le trifluoracétate de méthyle, le trifluoracétate d'éthyle, le trifluoracétate de propyle, le trifluoracétate de butyle, l'acétate de tertio-butyle, le propionate de tertio-butyle, le benzoate d'isopropyle, le benzoate de tertio-butyle, le 4-nitrobenzoate de méthyle ou le pentafluorobenzoate de méthyle,
$CF_3$-C(O)-O-$Si(CH_3)_3$, $CF_3$-C(O)-O-$Si(CH_3)_2$(t-$C_4H_9$), $CF_3$-C(O)-O-$Si(C_2H_5)_2$(i-$C_3H_7$), $CF_3$-C(O)-O-$Si(C_2H_5)_3$, $CF_3$-C(O)-O-Si(i-$C_3H_7)_3$, $CF_3$-C(O)-O-Si-(n-$C_4H_9)_3$, $CH_3$-C(O)-O-$Si(CH_3)_3$, $CH_3$-C(O)-O-$Si(CH_3)_2$(n-$C_4H_9$), $(C_2H_5)$-C(O)-O-Si-$(CH_3)_3$, $(C_2H_5)$-C(O)-O-$Si(C_2H_5)_3$, $C_2F_5$-C(O)-O-$Si(CH_3)_3$, $C_4F_9$-C(O)-O-$Si(CH_3)_3$, $C_4F_9$-C (O)-O-$Si(C_2H_5)_3$, $C_4F_9$-C(O)-O-$Si(CH_3)_2$(n-$C_4H_9$), $C_4F_9$-C(O)-O-$Si(CH_3)$(n-$C_4H_9)_2$, $C_8F_{17}$-C(O)-O-$Si(CH_3)_3$, $C_6H_5$-C(O)-O-$Si(CH_3)_3$, (p-$NO_2$)$C_6H_4$-C(O)-O-$Si(CH_3)_3$ ou ($C_6F_5$-C(O)-O-$Si(CH_3)_3$)
à des températures comprises entre 10° et 30°C, sans utilisation de solvant.

**2.** Utilisation du procédé selon la revendication 1, pour la purification de liquides ioniques ayant des anions alkyl- ou arylsulfonate ou des anions alkyl- ou arylcarboxylate qui sont contaminés par des halogénures d'onium.

**3.** Trifluorométhanesulfonates de formule (11)

$$HétN^+ [CF_3SO_3]^- \quad (11),$$

où
$HétN^+$ désigne un cation hétérocyclique choisi parmi le groupe

imidazolium

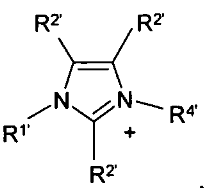

,

1H-pyrazolium

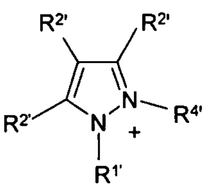

,

3H-pyrazolium

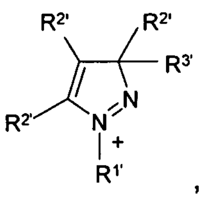

,

4H-pyrazolium

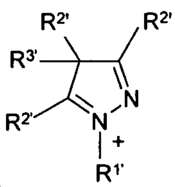

,

1-pyrazolinium

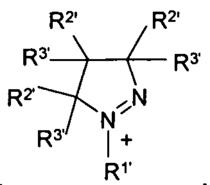

,

2-pyrazolinium

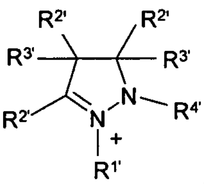

,

3-pyrazolinium

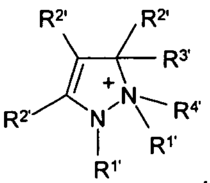

,

2,3-dihydroimidazolinium

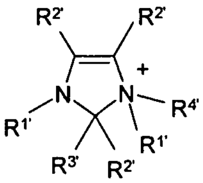

,

4,5-dihydroimidazolinium

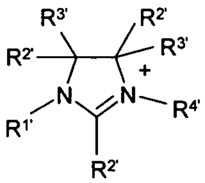

,

2,5-dihydroimidazolinium

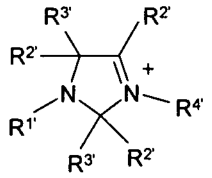

,

pyrrolidinium

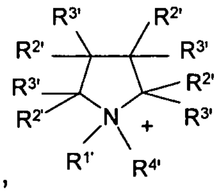

,

1,2,4-triazolium

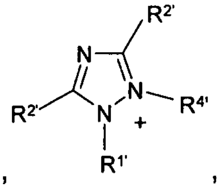

,

1,2,4-triazolium

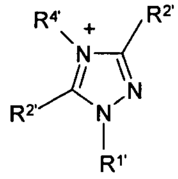

,

1,2,3-triazolium

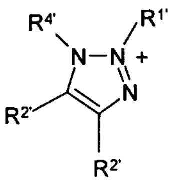

,

1,2,3-triazolium

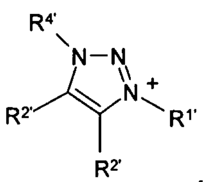

,

pyridinium

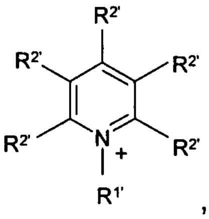

,

pyridazinium

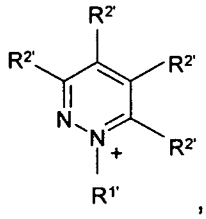

,

pyrimidinium

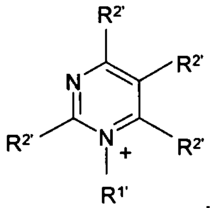

,

pipéridinium

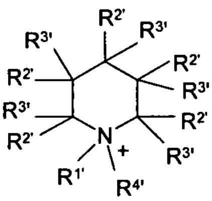

,

morpholinium

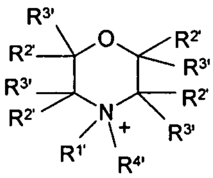

,

pyrazinium

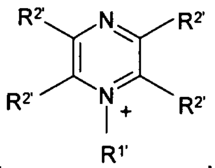

,

thiazolium

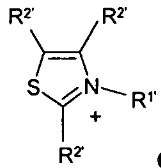

ou

oxazolium

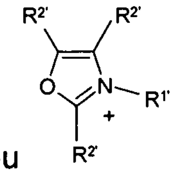

où
$R^{1'}$ ou $R^{4'}$ désigne CN et les autres substituants
$R^{1'}$ à $R^{4'}$ chacun, indépendamment les uns des autres, désignent hydrogène,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
dialkylamino ayant des groupements alkyle ayant 1-4 atomes de C, mais qui n'est pas lié à l'hétéroatome de l'hétérocycle,
cycloalkyle saturé, partiellement ou totalement insaturé ayant 3-7 atomes de C, pouvant être substitué par des groupements alkyle ayant 1-6 atomes de C,
ou aryl-$C_1$-$C_6$-alkyle,
où un ou plusieurs substituants $R^{2'}$ et $R^{3'}$ peuvent être partiellement ou totalement substitués par -F,
mais où $R^{1'}$ et $R^{4'}$ ne sont pas simultanément CN,
et où, dans les substituants $R^{1'}$ à $R^{4'}$, un ou deux atomes de carbone non adjacents qui ne sont pas liés directement à l'hétéroatome et qui ne sont pas en position ω peuvent être remplacés par des atomes et/ou des groupes d'atomes choisis parmi le groupe -O-, -S-, -S(O)- ou -$SO_2$-.

**4.** Utilisation des composés de formule (11) comme réactif de cyanation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2585979 A


### In der Beschreibung aufgeführte Nicht-Patentliteratur


- **RICHARD C. LAROCK.** Comprehensive Organic Transformations. Wiley-VCH, 1999
- Ionic Liquids in Synthesis. Wiley-VCH, 2003
- **HOUBEN-WEYL.** Methoden der organischen Chemie. Georg-Thieme-Verlag